# EUROPEAN PATENT APPLICATION

(11) **EP 1 560 133 A1**
(43) Date of publication of application: **03.08.2005**
(21) Application number: 03748737.8
(22) Date of filing: 07.10.2003
(51) Int. Cl.: G06F 17/50

(54) **THREE-DIMENSIONAL STRUCTURAL ACTIVITY CORRELATION METHOD**

(30) Priority: 07.10.2002 JP 2002293383
(71) Applicant: Nippon Zoki Pharmaceutical Co. Ltd., Osaka-shi, Osaka 541-0046 (JP)
(72) Inventor: KOTANI, T., Kato-gun, Hyogo 673-1461 (JP); HIGASHIURA, K., Kato-gun, Hyogo 673-1461 (JP)
(74) Representative: Manitz, Finsterwald & Partner GbR
(86) International application number: PCT/JP2003/012810
(87) International publication number: WO 2004/031999

(57) **Abstract**

A three-dimensional quantitative structure-activity relationship method has process B1 of calculating the coordinates of the respective atoms contained in the plural molecules thus superposed in the virtual space, process B2 of calculating interatomic distances between each atom and other atoms and identifying the shortest interatomic distance among thus calculated interatomic distances and two atoms constituting the shortest interatomic distance; process B3 of deleting the two atoms having the shortest interatomic distance from the three-dimensional space and generating an atom which represents the two atoms in the weighted average coordinates of the two atoms to delete, when the shortest interatomic distance thus calculated is equal to or smaller than a predetermined threshold value; process B4 of returning to the second process B2 after the third process B3 and executing the second process B2 including the atoms formed during the third process B3; and process B5 of terminating the process B when the shortest interatomic distance thus calculated is exceeds the predetermined threshold. This method enables strikingly reducing the memory zone and amount of computation required for 3D QSAR analysis.

## Description

### FIELD OF THE INVENTION

The present invention relates to a three-dimensional quantitative structure-activity relationship (3D QSAR) method and a program for quantitatively analyzing a relationship between the three-dimensional structure and the biological activity of a compound utilizing a statistical approach.

### BACKGROUND OF ART

As a method of designing a drug molecule having a desired biological activity, logical molecule design methods utilizing three-dimensional quantitative structure-activity relationship (3D QSAR) analysis, pharmacophore mapping and the like are used. Where these methods are used, statistical processing is performed utilizing a PLS (partial least square of latent valuables) method, a neural net (NN) method, genetic algorithm (GA) or the like after superposition of known drugs one atop the other within a virtual space in accordance with a proper rule, thereby extracting characteristics between various parameters such as biological activity, hydrophobicity and electrostatic interactions. The result can be displayed as graphics, and it is therefore possible to visually recognize portions (functional groups, three-dimensional structures) contributing to the activity inside a molecular structure and use them as a clue for molecular designing. It is further possible to apply this to prediction of the activity of a newly designed molecule.

Molecular superposition which is the first step of 3D QSAR analysis has heretofore used an approach of superposing presumably corresponding atoms with each other or functional groups with each other between plural molecules to be compared or an approach of sequentially searching for the best superposition by means of an evaluation function (molecular similarity). However, although completing superposition in a short period of time, the approach of superposing atoms with each other or functional groups with each other has a disadvantage that researcher's subject is inevitably reflected. For instance, subjective superposition of different molecules one atop the other by a researcher may result in something which is quite different from superposition of conformations in which actual molecules interact with receptor proteins. Meanwhile, an approach of automatically extracting functional groups using a computer still has a problem that selection of the types and number of functional groups to be superposed is susceptible to the arbitrariness of software dependency, researcher's subject, etc. Although an approach using an evaluation function is ideal as a molecular superposition procedure per se, this approach has a flaw that computation takes time. Noting this, the inventors of the present invention have discussed development of a molecular superposition method which is faster and non-arbitrary, and invented and reported a method which a standard PC can execute at a computation speed which is 100 through 1000 times as fast as that of conventional methods (Kotani, T.; Higashiura, I. Rapid evaluation of molecular shape similarity index using pairwise calculation of the nearest atomic distances. J. Chem. Inf. Comput. Sci. 2002, 42, 58-63.).

It is a 3D QSAR program that is needed after superposition of molecules. However, there are only few integrated molecular design packages for 3D QSAR that can be executed on a standard PC, and further, since such a 3D QSAR analysis method is available as a dedicated module of an integrated molecular design package and therefore it is not possible to obtain only this. In addition, most 3D QSAR analysis methods are very often run on expensive general purpose computers, workstations, etc. This makes it difficult for a synthetic chemist to conveniently perform 3D QSAR while conducting a test and apply this to optimization of a target compound. Plural QSAR analysis methods proposed so far will now be described in specific details.

### (1) Classical QSAR method:

For analysis, a classical QSAR method, typically the Fujita-Hansch method, uses parameters such as a hydrophobic parameter n, an electrostatic parameter σ and a three-dimensional parameter Es assigned to a functional group, and by means of a statistical method such as multiple regression analysis (MRA), extracts a physiochemical property contributing to the activity, and applies this to drug discovery. Hence, while realizing analysis of only a group of compounds having relatively similar skeletons, the method has a disadvantage that QSAR analysis can not be made on a group of compounds having functional groups to which parameters have not been assigned. The greatest defect is that this method is not applicable to three-dimensional QSAR analysis.

### (2) Comparative Molecular Field Analysis (CoMFA) method:

CoMFA developed by Cramer et al. (Cramer III, R. D.; Patterson, D. E.; Bunce, J. D. Comparative Molecular Field Analysis (CoMFA). 1. Effect of Shape on Binding of Steroids to Carrier Proteins. J. Am. Chem. Soc. 1988, 110, 5959-5967) aims at QSAR analysis noting a "field" surrounding a drug molecule. CoMFA analysis assumes that a difference between the structures of molecules appears as a difference between "fields" around the molecules and that this influences a biological activity value. Hence, for the purpose of properly reflecting a structure difference in data, the molecular structures must be appropriately superposed each other, which is similar to other 3D QSAR methods than CoMFA. After superposition, a box enclosing the superposed molecules is then considered, and inside the box, a few thousands lattice points are created which are apart 1 or 2 angstroms from each other. Following this, an imaginary sp³ carbon atom having a charge of +1 is inserted at the position of each lattice point, the steric and the electrostatic potentials between each drug molecule and each sp³ carbon atom thus inserted are calculated and used as three-dimensional structure descriptors for each drug molecule (CoMFA fields).

During calculation of CoMFA fields, the steric interactions are calculated by the Lennard-Jones formula and the electrostatic interactions are calculated using Coulomb potentials. CoMFA fields are calculated for each one of the superposed molecules are calculated, and used as three-dimensional structure descriptors for each molecule to thereby statistically analyze the relationship with activity values. A PLS (Partial Least Square) method is used for statistical analysis, and a calculated activity prediction formula is indicative of properties demanded from the drug molecules and can be expressed as three-dimensional graphics. It is possible to show in an easy-to-follow manner, as computer graphics, a guideline regarding which substitutional groups having which properties should be sterically and electrostatically inserted in which portions of the molecules or how substitutional groups should be deleted to obtain a more active compound.

Since no parameter indicative of hydrophobic interactions is available for CoMFA, Kellogg et al. have invented a parameter called HINT and applied it to CoMFA analysis (Kellogg, G. E.; Semus, S. F.; Abraham, D. J. HINT: a new method of empirical hydrophobic field calculation for CoMFA. J. Comput. Aided Mol. Des. 1991, 5, 545-552, Kellogg, G. E.; Abraham, D. J. Hydrophobicity: is LogP(o/w) more than the sum of its parts? Eur. J. Med. Chem. 2000, 35, 651-661.).

### (3) Comparative Molecular Similarity Analysis (CoMSIA) method:

Klebe et al. have reported CoMSIA, as a 3D QSAR calculation method which is on extension of CoMFA (Klebe, G.; Abraham, U.; Mietzner, T. Molecular similarity indices in a comparative analysis (CoMSIA) of drug molecules to correlate and predict their biological activity. J. Med. Chem. 1994, 37, 4130-4146., Klebe G. Comparative Molecular Similarity Indices Analysis: CoMSIA. Perspect. Drug Discov. Design 1998, 12/13/14, 87-104, Klebe, G.; Abraham, U. Comparative molecular similarity index analysis (CoMSIA) to study hydrogen-bonding properties and to score combinatorial libraries. J. Comput. Aided Mol. Des. 1999, 13, 1-10.).

A similarity index is used for calculation of "fields" and similar calculation to that of CoMFA, whereas CoMFA requires calculation using steric potentials, electrostatic potentials and a few additional fields for CoMFA calculation.

CoMSIA presents an improvement over a few disadvantages of CoMFA. To be more specific, since Lennard-Jones potentials used in CoMFA are acutely steep in the vicinity of the van der Waals surface, the potential energy abruptly changes at a lattice point near the surface of the molecular. This may lead to a largely different result, owing to a small change of the conformation of the molecules. Further, in the case of Lennard-Jones potentials or Coulomb potentials, a lattice point on an atom becomes a singularity and hence has a meaningless value such as infinity and infinitesimal, it is necessary to cut off the potential energy. In addition, since the gradient of the potential is different between a Lennard-Jones potential and a Coulomb potential, there is a disadvantage that the distances from a molecule which is cut off are different. In short, cut-off must be at different distances from the molecule between these potentials, and it is therefore predicted that the rates of contribution will not be accurately reflected. CoMSIA, noting this, demands use of the SEAL function, which is used as a molecular superposition method, to calculate steric fields and electrostatic fields (As for "SEAL function", see Klebe, G.; Mietzner, T.; Weber, F. Different approaches toward an automatic structural alignment of drug molecules: applications to sterol mimics, thrombin and thermolysin inhibitors. J. Comput. Aided Mol. Des. 1994, 8, 751-778.). In relation to the SEAL function, applications of a hydrogen-bonding donor field, a hydrogen-bonding acceptor field and a hydrophobic field have been reported. Using a Gaussian evaluation formula, SEAL does not result in creation of singularities, which is a problem with CoMFA, and does not necessitate cut-off.

On the contrary, CoMFA and CoMSIA are known to influence the result of QSAR analysis because of arbitrary creation of lattice points. Although there are MFA methods which improve creation of lattice points to overcome this disadvantage, any one of these methods requires reduction of the spaces between lattice points to increase the accuracy of calculation, and in some cases, necessitates several thousands or more lattice points. While a greatly increased number of lattice points are necessary to obtain an accurate 3D QSAR analysis result, the amount of computing also increases, which suggests that the reliability of 3D QSAR is influenced to a large extent by the capability of a computer.

### (4) Hypothetical Active Site Lattice (HASL) method:

As for the HASL method, unlike CoMFA and CoMSIA, HASL developed by Doweyko is a method according to which lattice points are created about 2 angstroms apart from each other in a region which is at or within the van der Waals radius of a molecule, the physiochemical properties of the molecules are assigned to the respective lattice points, and unique fitting is executed (Doweyko, A. M. Three-dimensional pharmacophores from binding data. J. Med. Chem. 1994, 37, 1769-1778, Guccione, S.; Doweyko, A. M.; Chen, H.; Barretta, G. U.; Balzano, F. 3D QSAR using 'multiconformer' alignment: the use of HASL in the analysis of 5-HTIA thienopyrimidinone ligands. J. Comput. Aided Mol. Des. 2000, 14, 647-657.). As compared with CoMFA, CoMSIA and MFA (available from Accelrys Inc.), HASL needs a dramatically smaller number of lattice points, about one hundred, which permits computation on a standard PC but yet has a similar problem to those with CoMFA, CoMSIA and the like in that creation of lattice points is still arbitrary. Further, there is only one type of HASL atoms available for HASL, and these can have a value of either +1, 0 or -1 owing to their physiochemical properties. As for a derivative for which the HASL atom type is not defined, it is not possible to conduct QSAR analysis.

### (5) Methods of superposing pharmacophores:

These are methods of 3D QSAR through evaluation of how much physiochemical properties, such as hydrogen bonds, electrostatic interactions and hydrophobic pockets, needed for onset of activity are present in a model, and to be specific, they are DISCO, Catalyst, Apex-3D, etc. However, although these computation methods are convenient and have been used for superposition of derivatives, these computation methods have a disadvantage that a result becomes different depending upon how physiochemical properties are defined. As for DISCO, see Martin, Y. C.; Bures, M. G.; Danaher, E. A.; Delazzer. J.; Lico, I.; Pavlik, P. A. A fast new approach to pharmacophore mapping and its application to dopaminergic and benzodiazepine agonists. J. Comput. Aided Mol. Des. 1993, 7, 83-102. As for Catalyst, see Greene, J.; Kahn, S.; Savoj, H.; Sprague, P.; Teig, S. Chemical Function Queries for 3D Database Search. J. Chem. Inf. Comput. Sci., 1994, 34, 1297-1308.

In summary, the conventional 3D QSAR methods have the following disadvantages.
(a) Since thousands lattice points need be created, the amount of computing increases, a large memory area is necessary and it is not possible to run 3D QSAR analysis on a standard PC.
(b) Depending upon how a compound under modeling is oriented relative to lattice points, a result may become different.
(c) Elimination of a singularity and cut-off is necessary.
(d) Some are difficult to assign the types of atoms, and the types of atoms are not assigned to some.

### SUMMARY OF THE INVENTION

A three-dimensional quantitative structure-activity relationship method according to the present invention comprises:
a process A of superposing plural molecules in a virtual space;
a process B of performing cluster analysis of the atomic coordinates of the plural molecules thus superposed in the virtual space and thereby generating represented points;
a process C of calculating interactions (steric interactions, electrostatic interactions and hydrophobic interactions for instance) between the respective atoms of the plural molecules thus superposed and the represented points; and
a process D of statistically analyzing the interactions.

In particular, the process B of cluster analysis further comprises:
a first process B1 of calculating the coordinates of the respective atoms contained in the plural molecules thus superposed in the virtual space;
a second process B2 of calculating interatomic distances between each atom and other atoms and identifying the shortest interatomic distance among thus calculated interatomic distances and two atoms constituting the shortest interatomic distance;
a third process B3 of deleting the two atoms having the shortest interatomic distance from the three-dimensional space and generating an atom which represents these two atoms in the weighted average coordinates of the two atoms to delete, when the calculated shortest interatomic distance is equal to or smaller than a predetermined threshold value;
a fourth process B4 of returning to the second process B2 after the third process B3 and executing the second process B2 including the atoms formed during the third process B3; and
a fifth process B5 of terminating the process B when the calculated shortest interatomic distance is exceeds the predetermined threshold.

According to other aspect of the present invention, in the three-dimensional quantitative structure-activity relationship method, the process B in particular further comprises:
a process B1 of, when the molecules thus superposed in the virtual space include a ring structure or functional group, generating an imaginary atom (pseudo-atom) at a position representing the ring structure or functional group;
a process B2 of calculating interatomic distances between each atom and other atoms as for all atoms in the virtual space including the imaginary atom and identifying the shortest interatomic distance among thus calculated interatomic distances and two atoms constituting the shortest interatomic distance;
a process B3 of deleting the two atoms having the shortest interatomic distance from the three-dimensional space and generating an atom which represents these two atoms in the weighted average coordinates of the two atoms to delete, when the calculated shortest interatomic distance is equal to or smaller than a predetermined threshold value;
a process B4 of returning to the second process B2 after the third process B3; and
a process B5 of terminating the process B when the calculated shortest interatomic distance is exceeds the predetermined threshold.

In this manner, where a pseudo-atom is generated as an imaginary point which represents a functional group, it is possible to decrease the number of the "atoms" used for computing, reduce the amount of computing needed for 3D QSAR analysis, and analyze faster and more conveniently. Whether to set a point which represents a functional group, where to set the point and the like may be determined appropriately depending upon the type of the functional group, parameters to use, etc. In other words, the point which represents the functional group can be set at the center of the functional group, a position which uses weighted average or arithmetic average considering the atomic weight, etc., and plural such points may be set. Further, in the event that molecules have a ring structure, a pseudo-atom may be set additionally at a position which represents the ring structure. In this case, unlike setting of a pseudo-atom for a functional group, the atoms constituting the ring structure are left a the pseudo-atom is additionally set. This permits consideration of characteristics of the ring portion of the molecules and discovery of a more preferable structure-activity relationship. The position at which the pseudo-atom is set may be properly determined in a similar manner to that for setting of a pseudo-atom which represents a functional group.

The present invention is directed also to a program for a three-dimensional quantitative structure-activity relationship method of extracting and visually displaying characteristics of a compound based on the atomic coordinates of plural molecules which are superposed in a virtual space on a computer, the program making a computer execute:
a process A of superposing plural molecules in a virtual space;
a process B of performing cluster analysis of the atomic coordinates of the plural molecules thus superposed in the virtual space and thereby generating represented points;
a process C of calculating interactions between the respective atoms of the plural molecules thus superposed and the represented points; and
a process D of statistically analyzing the interactions.

In particular, the process B of cluster analysis comprises:
a first process B1 of calculating the coordinates of the respective atoms contained in the plural molecules thus superposed in the virtual space;
a second process B2 of calculating interatomic distances between each atom and other atoms and identifying the shortest interatomic distance among thus calculated interatomic distances and two atoms constituting the shortest interatomic distance;
a third process B3 of deleting the two atoms having the shortest interatomic distance from the three-dimensional space and generating an atom which represents these two atoms in the weighted average coordinates of the two atoms to delete, when the calculated shortest interatomic distance is equal to or smaller than a predetermined threshold value;
a fourth process B4 of returning to the second process B2 after the third process B3 and executing the second process B2 including the atoms formed during the third process B3; and
a fifth process B5 of terminating the process B when the calculated shortest interatomic distance is exceeds the predetermined threshold.

According to other aspect of the present invention, during the process B of cluster analysis, the program achieves execution of:
a first process B1 of, when the molecules thus superposed in the virtual space include a ring structure or functional group, generating an imaginary atom at a position which represents the ring structure or functional group when needed;
a second process B2 of, as for all atoms in the virtual space including the imaginary atom, calculating interatomic distances with other atoms and identifying the shortest interatomic distance among thus calculated interatomic distances and two atoms constituting the shortest interatomic distance;
a third process B3 of deleting the two atoms having the shortest interatomic distance from the three-dimensional space and generating an atom which represents these two atoms in the weighted average coordinates of the two atoms to delete, when the calculated shortest interatomic distance is equal to or smaller than a predetermined threshold value;
a fourth process B4 of returning to the second process B2 after the third process B3 and executing the second process B2 including the atoms formed during the third process B3; and
a fifth process B5 of terminating the process B when the calculated shortest interatomic distance is exceeds the predetermined threshold.

When such a three-dimensional quantitative structure-activity relationship method and the program for the same are used, instead of generating lattice points around molecules as in CoMFA, CoMSIA and MFA, represented points for calculation of interactions are generated inside the molecules, and hence, the number of points needed for computing is greatly reduced. This remarkably reduces the amount of computing and a memory area required for 3D QSAR analysis.

In addition, the atomic coordinates of the molecules are determined through cluster analysis referring to a certain threshold value as an index, instead of using lattice points as points for calculation of interactions. In other words, the atomic coordinates of the molecules which are used for calculation and the coordinates of a pseudo-atom which is set when needed are extracted, and such xyz coordinates are used which are obtained by weighted averaging of the xyz coordinates of atoms and pseudo-atoms which are equal to or smaller than a predetermined threshold value. This ensures the same result no matter how molecules are oriented relative to the xyz axes. Further, since many coordinate points are formed where the structure changes largely, it is expected that the spaces between the coordinate points are narrow in a region which presumably contributes to the activity, whereas in a region which presumably does not makes a great contribution to the activity, the spaces between the coordinate points are wide.

Further, use of an evaluation formula, a Gaussian evaluation formula or indicator coefficients in a rapid molecular superposition approach for calculation of interactions makes it possible to avoid singularities, cut-off, etc.

Moreover, it is possible to handle all atom types when the van der Waals radius, a partial charge of an electron or the like may each be used alone or an indicator coefficient derived from these values may be used as each one of a steric parameter and an electrostatic parameter. In addition, those which are already known may be applied as a hydrophobic parameter, a hydrogen-bonding parameter, etc.

### BRIEF DESCRIPTION OF THE DRAWINGS

Fig. 1 is a flow chart which outlines the three-dimensional quantitative structure-activity relationship method according to the present invention;
Fig. 2 is a diagram showing the details of cluster analysis (STEP 2) shown in Fig. 1;
Fig. 3 is a diagram showing a calculation process in CoMFA;
Fig. 4 is a diagram showing a compound set of steroid derivatives used for superposition;
Fig. 5 is a diagram showing analysis results (steric interactions) of CoMSIA;
Fig. 6 is a diagram showing analysis results (electrostatic interactions) of CoMSIA;
Fig. 7 is a diagram showing represented points which are generated based on the atomic coordinates of superposed molecules;
Fig. 8 is a diagram showing represented points which are generated by adding new points (pseudo-atoms) in central portions of rings;
Fig. 9 is a graph showing a result of PLS analysis using a rapid superposition method;
Fig. 10 is a diagram showing a result of PLS analysis using a rapid superposition method;
Fig. 11 is a graph showing a result of PLS analysis using the SEAL evaluation formula;
Fig. 12 is a diagram showing the contribution of a steric term, on a result of PLS analysis using the SEAL evaluation formula;
Fig. 13 is a diagram showing the contribution of an electrostatic term, on a result of PLS analysis using the SEAL evaluation formula;
Fig. 14 is a graph showing a result of PLS analysis using the molecular similarity evaluation formula developed by Good et al.;
Fig. 15 is a diagram showing the contribution of a steric term, on a result of PLS analysis using the molecular similarity evaluation formula developed by Good et al.;
Fig. 16 is a diagram showing the contribution of an electrostatic term, on a result of PLS analysis using the molecular similarity evaluation formula developed by Good et al.;
Fig. 17 is a graph showing a result of PLS analysis using an indicator variable;
Fig. 18 is a diagram visualizing the contribution of a steric term, on a result of PLS analysis using an indicator variable;
Fig. 19 is a diagram showing the contribution of an electrostatic term, on a result of PLS analysis using an indicator variable;
Fig. 20 is a graph showing a result of PLS analysis using the SEAL evaluation formula which is obtained with an atom inserted at the center of a ring;
Fig. 21 is a diagram showing the contribution of a steric term, on a result of PLS analysis using the SEAL evaluation formula which is obtained with an atom inserted at the center of a ring;
Fig. 22 is a diagram showing the contribution of an electrostatic term, on a result of PLS analysis using the SEAL evaluation formula which is obtained with an atom inserted at the center of a ring
Fig. 23 is a graph showing a result of PLS analysis using the molecular similarity evaluation formula developed by Good et al. which is obtained with an atom inserted at the center of a ring;
Fig. 24 is a diagram showing the contribution of a steric term, on a result of PLS analysis using the molecular similarity evaluation formula developed by Good et al. which is obtained with an atom inserted at the center of a ring;
Fig. 25 is a diagram showing the contribution of an electrostatic term, on a result of PLS analysis using the molecular similarity evaluation formula developed by Good et al. which is obtained with an atom inserted at the center of a ring;
Fig. 26 is a diagram showing the contribution of a hydrophobic term, on a result of PLS analysis applying a hydrophobic parameter for the SEAL method which is obtained from a Gaussian evaluation formula;
Fig. 27 is a diagram showing the contribution of a hydrophobic term, on a result of PLS analysis obtained from an indicator variable applying a hydrophobic parameter for the SEAL method;
Fig. 28 is a diagram showing the contribution of a hydrophobic term, on a result of PLS analysis obtained from a Gaussian evaluation formula applying a hydrophobic parameter for the FLEXS method;
Fig. 29 is a diagram showing the contribution of a hydrophobic term, on a result of PLS analysis obtained from an indicator variable applying a hydrophobic parameter for the FLEXS method;
Fig. 30 is a diagram showing the contribution of an HASL parameter, on a result of PLS analysis obtained from a Gaussian evaluation formula applying the HASL parameter;
Fig. 31 is a diagram showing the contribution of an HASL parameter, on a result of PLS analysis obtained from an indicator variable applying the HASL parameter;
Fig. 32 is a diagram showing the contribution of a steric term, on a result of PLS analysis obtained using the Audry formula as an attenuation function;
Fig. 33 is a diagram showing the contribution of a steric term, on a result of PLS analysis obtained using the Fauchère formula as an attenuation function;
Fig. 34 is a diagram showing the contribution of a steric term, on a result of PLS analysis obtained using the modified Fauchère formula as an attenuation function;
Fig. 35 is a diagram showing the contribution of a steric term, on a result of PLS analysis from the SEAL-type Gaussian function;
Fig. 36 is a diagram showing the contribution of a steric term, on a result of PLS analysis obtained from an indicator variable;
Fig. 37 is a diagram showing the contribution of an electrostatic term, on a result of PLS analysis obtained using the Audry formula as an attenuation function;
Fig. 38 is a diagram showing the contribution of an electrostatic term, on a result of PLS analysis obtained using the Fauchère formula as an attenuation function;
Fig. 39 is a diagram showing the contribution of an electrostatic term, on a result of PLS analysis obtained using the modified Fauchère formula as an attenuation function;
Fig. 40 is a diagram showing the contribution of an electrostatic term, on a result of PLS analysis obtained by a SEAL-type Gaussian function;
Fig. 41 is a diagram showing the contribution of an electrostatic term, on a result of PLS analysis obtained from an indicator variable;
Fig. 42 is a diagram showing the contribution of a hydrophobic term, on a result of PLS analysis obtained using the Fauchère formula as an attenuation function while applying an FLEXS parameter;
Fig. 43 is a diagram showing the contribution of a hydrophobic term, on a result of PLS analysis obtained using the modified Fauchère formula as an attenuation function while applying an FLEXS parameter;
Fig. 44 is a diagram showing the contribution of a hydrophobic term, on a result of PLS analysis obtained using a SEAL-type Gaussian function as an attenuation function while applying an FLEXS parameter;
Fig. 45 is a diagram showing the contribution of a hydrophobic term, on a result of PLS analysis obtained using the Audry formula as an attenuation function while applying the AlogP parameter;
Fig. 46 is a diagram showing the contribution of a hydrophobic term, on a result of PLS analysis obtained using the Fauchère formula as an attenuation function while applying the AlogP parameter;
Fig. 47 is a diagram showing the contribution of a hydrophobic term, on a result of PLS analysis obtained using the modified Fauchère formula as an attenuation function while applying the AlogP parameter;
Fig. 48 is a diagram showing the contribution of an electrostatic term, on a 3D QSAR result of COX-2;
Fig. 49 is a diagram showing the contribution of a steric term, on a 3D QSAR result of COX-2; and
Fig. 50 is a diagram showing the contribution of a hydrophobic term, on a 3D QSAR result of COX-2.

### BEST MODE FOR IMPLEMENTING THE INVENTION

A three-dimensional quantitative structure-activity relationship method using the computation technique according to the present invention will now be described with reference to the associated drawings. Although not shown in the drawings, the structure-activity relationship method in the present invention is run on a computer and realized as a computer executes a program which is written in a proper programming language. Further, the program is recorded on various types of known recording media such as a CD-ROM or provided on the Internet, a telecommunication line such as a telephone line.

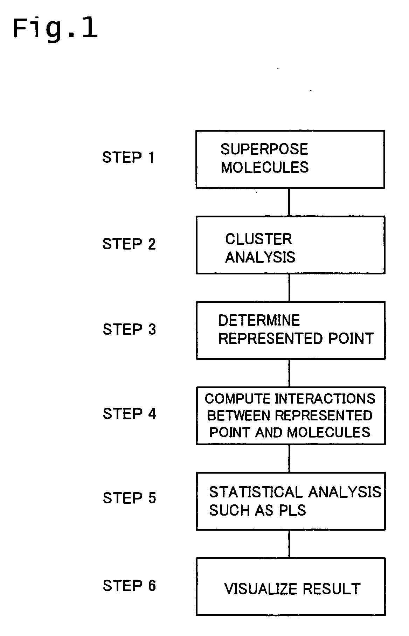

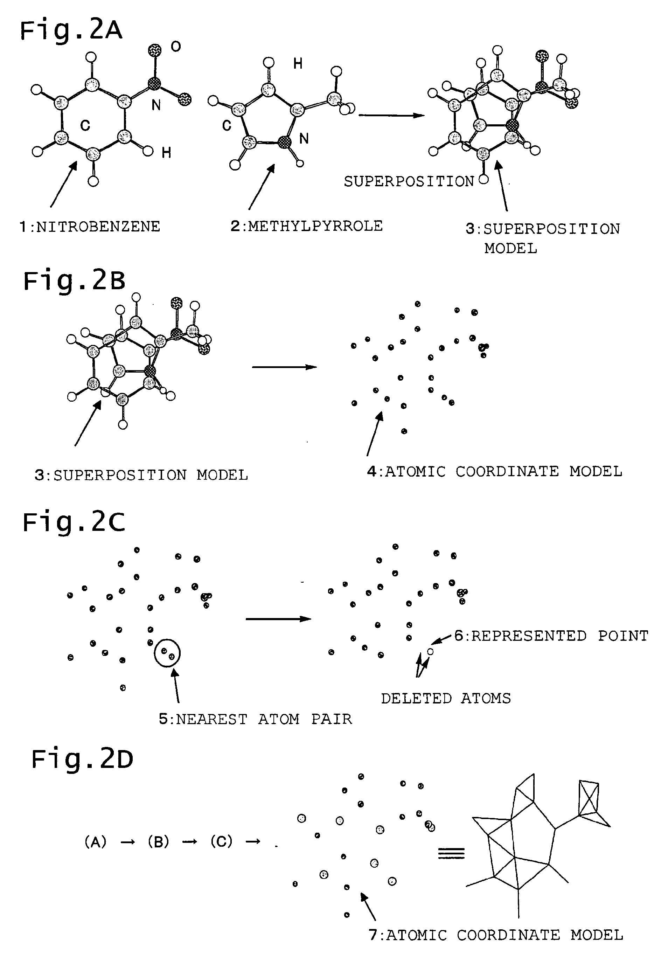

Fig. 1 shows the overview process of the structure-activity relationship method according to the present invention. As shown in Fig. 1, this structure-activity relationship method requires superposition of plural molecules to be analyzed within a virtual space (xyz coordinate space) (STEP 1). For instance, for analysis of nitrobenzene and methylpyrrole, as shown in Fig. 2(A), three-dimensional structure data (data including the three-dimensional coordinates of plural atoms contained in each molecule) on the molecules of both nitrobenzene 1 and methylpyrrole 2 are acquired, the molecules of the both are superposed one atop the other within a virtual three-dimensional space using this structure data, and a superposition model 3 is developed. While two molecules are superposed in the drawing for simplicity of description, any desired number of molecules may be superposed.

Referring back to Fig. 1, cluster analysis is carried out on thus superposed molecules (STEP 2). During this cluster analysis, first, the atomic coordinates of the two molecules superposed within the virtual space are extracted. As shown in Fig. 2(B) for example, the coordinates of atoms contained in the superposed two molecules (nitrobenzene and methylpyrrole) alone are extracted and an atomic coordinate model 4 is created. Next, the distances (spatial distances) between each atom and other atoms are calculated and a pair of atoms having the shortest interatomic distance (nearest atom pair 5) is identified. Following this, the shortest interatomic distance of the nearest atom pair 5 is compared with a preset threshold value. The threshold value may be any desired value, e.g., 0.75 angstrom. When the shortest interatomic distance found as a result of this to be equal to or smaller than the threshold value (or smaller than the threshold value), the two atoms forming the nearest atom pair 5 are removed from the virtual space, and the weighted average coordinates of the coordinates of these two atoms (intermediate coordinates of the two atoms) are calculated, and a representative atom 6 is generated in the weighted average coordinates, as shown in Fig. 2(C) (STEP 3). For distinction from atoms other than the representative atom during later computing, weight corresponding to the number of atoms constituting the representative atom is preferably allocated to the representative atom 6.

To be noted is that when the molecules superposed within the virtual space have a functional group, a pseudo-atom may be hypothetically generated at a position representing the functional group, in which case the number of "atoms" used for computing is smaller, the amount of computing needed for 3D QSAR analysis is therefore smaller and the analysis is faster and more convenient. Whether to set a point which represents a functional group, where to set the point and the like may be determined appropriately depending upon the type of the functional group, parameters to use, etc. In other words, the point which represents the functional group can be set at the center of the functional group, a position which uses weighted average or arithmetic average considering the atomic weight, etc., and plural such points may be set. Further, in the event that molecules have a ring structure, a pseudo-atom may be set additionally at a position which represents the ring structure. In this case, unlike setting of a pseudo-atom for a functional group, the atoms constituting the ring structure are left and a pseudo-atom is additionally set. This permits consideration of characteristics of the ring portion of the molecules and discovery of a more favorable structure-activity relationship. The position at which the pseudo-atom is set may be properly determined in a similar manner to that for setting of a pseudo-atom which represents a functional group.

Next, viewing the newly generated representative atom 6 as one atom, the distances between each atom and other atoms are calculated in a similar fashion to the above, and when the shortest interatomic distance is equal to or smaller than the threshold value (or smaller than the threshold value), two atoms which are at the shortest interatomic distance are deleted from the virtual space and a new representative atom 6 is generated.

Generation of a representative atom 6 is repeated until the shortest interatomic distance reaches or exceeds the threshold value, and as shown in Fig. 2(D), an atomic model 7 is created. The coordinates of the representative atom 6 generated in the manner above will be referred to as a "represented point".

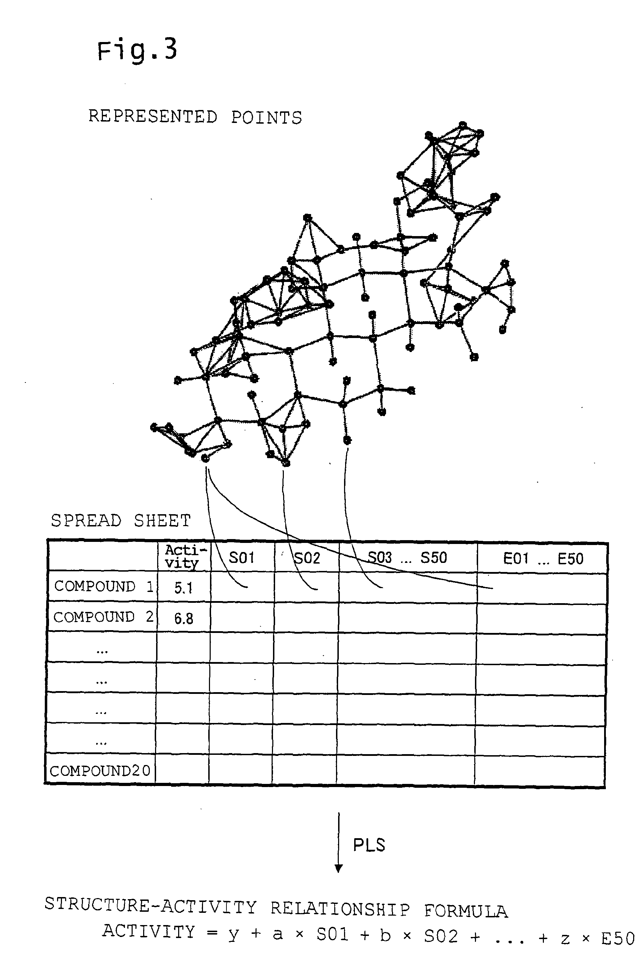

Referring back to Fig. 1, interactions between the represented point and the molecules are calculated using an appropriate evaluation function after cluster analysis (STEP 4). During this computing, as shown in Fig. 3, steric interactions, electrostatic interactions and hydrophobic interactions between the represented point and each atom of the superposed plural molecules are calculated. Steric interactions and electrostatic interactions are calculated from a Gaussian formula for instance. The molecular similarity evaluation method which the inventors of the present invention have proposed in Kotani, T.; Higashiura, K. Rapid evaluation of molecular shape similarity index using pairwise calculation of the nearest atomic distances. J. Chem. Inf. Comput. Sci., 2002, 42, 58-63. can be preferably applied to steric interactions. Parameters for FLEXS (Lemmen, C.; Lengauer, T.; Klebe, G. FLEXS: a method for fast flexible ligand superposition. J. Med. Chem. 1998, 41, 4502-4520) can be preferably applied to hydrophobic interactions.

Following this, thus obtained interaction results are analyzed by PLS analysis (STEP 5) and data is visualized (STEP 6), which is similar to CoMFA and CoMSIA. CoMFA and the like which are conventional 3D GSAR approaches require handling the values of potentials calculated at as many as hundreds through thousands (depending upon the sizes of molecules) lattice points as structure descriptors (explanatory variables) for the respective molecules, and to this end, the PLS method, a type of regression analysis, is used. According to the PLS method, a value called a "component" correlated with an object variable (such as a pharmacological activity value) is extracted from among a number of descriptors, and a regression equation is formed. The "component" is very similar in nature to a principal component which is computed in principal component analysis, and where plural components are extracted, they are orthogonal to each other. Due to this, it is possible to frame an activity prediction formula from data containing a very large number of variables, e.g., CoMFA data. The number of PLS components is determined by the reliability evaluation method called "Leave-one-out" method, and with the number of components necessary to form the most reliable activity prediction formula, an activity prediction formula is made.

### EXAMPLE

### I. MODEL USED FOR COMPUTING

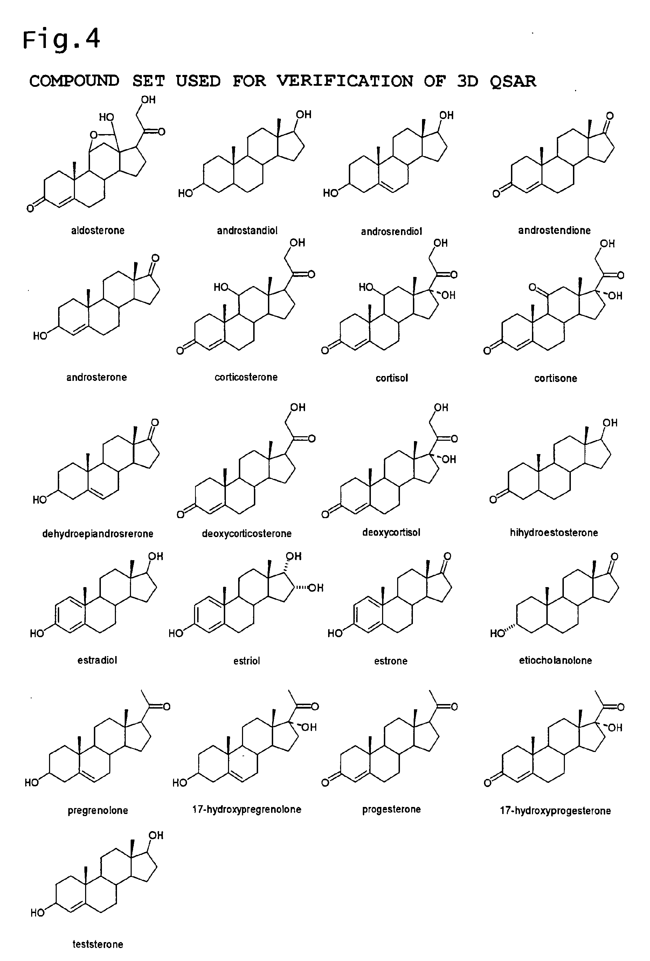

To study the usefulness of the 3D QSAR method according to the present invention, 3D QSAR analysis was conducted using the structure-activity relationships regarding steroid derivatives disclosed by Cramer et al. in their presentation on CoMFA, which ever since then have become the benchmark for many 3D QSAR analysis software, as a model. Fig. 4 shows the steroid derivatives used for superposition and Table 1 shows the binding activity of each compound relative to human corticosteroid-binding globulin.

**Table 1**

| Binding affinity to human corticosteroid-binding globulins | |
|---|---|
| Compound | Binding affinity to human corticosteroid-binding globulins (CBG) |
| aldosterone | -6.279 |
| androstandiol | -5.000 |
| androstenediol | -5.000 |
| androstenedione | -5.763 |
| androsterone | -5.613 |
| corticosterone | -7.881 |
| cortisol | -7.881 |
| cortisone | -6.892 |
| dehydroepiandrosterone | -5.000 |
| deoxycorticosterone | -7.653 |
| deoxycortisole | -7.881 |
| dihydrotestosterone | -5.919 |
| estradiol | -5.000 |
| estriol | -5.000 |
| estrone | -5.000 |
| etiocholanolone | -5.255 |
| pregnenolone | -5.255 |
| hydroxy pregnenolone | -5.000 |
| progesterone | -7.380 |
| hydroxy progesterone | -7.740 |
| teststerone | -6.724 |

As the xyz coordinates, partial charges and the like of the steroid molecules, the files containing the training sets used in the CoMFA report were used directly. As the xyz coordinates, partial charges and the like of COX-2 inhibitors, the data according to Liu et al. was used directly. Cygwin 1.3.2 on Windows NT 4.0 was used for all computing, and a program was created in Fortran, C and Tcl/tk. SAMPLS (QCPE#650) (Bush, B. L.; Nachbar, R. B., Jr. Sample-distance partial least squares: PLS optimized for many variables, with application to CoMFA. J. Comput. Aided Mol. Des. 1993, 7, 587-619.) was used for PLS calculation, and WebLab ViewerLite 4.0 (available from Accerlys) was used for visualization of the computing results.

Further, as a way to confirm the validity of this method, 3D QSAR analysis was conducted using the cyclooxygenase (COX-2) inhibitors reported by Liu et al. (Liu, H.; Huang, X.; Shen, J.; Luo, X.; Li, M.; Xiong, B.; Chen, G.; Yang, Y.; Jiang, H.; Chen, K. Inhibitory Mode of 1,5-Diarylpyrazole Derivatives Against Cyclooxygenase-2 and Cyclooxygenase-1: Molecular Docking and 3D QSAR Analyses. J. Med. Chem. 2002, 45, 4816-4827). For this analysis, the binding conformations of 1,5-diarylpyrazole derivatives and COX-2 which Liu et al. calculated using AutoDock were used.

### II. RESULTS OBTAINED WITH CONVENTIONAL ANALYSIS METHODS (EXAMPLE FOR COMPARISON)

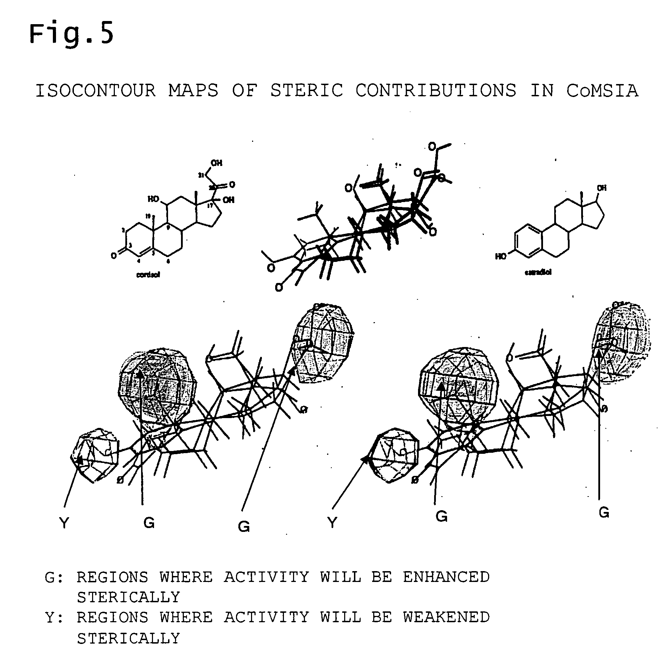

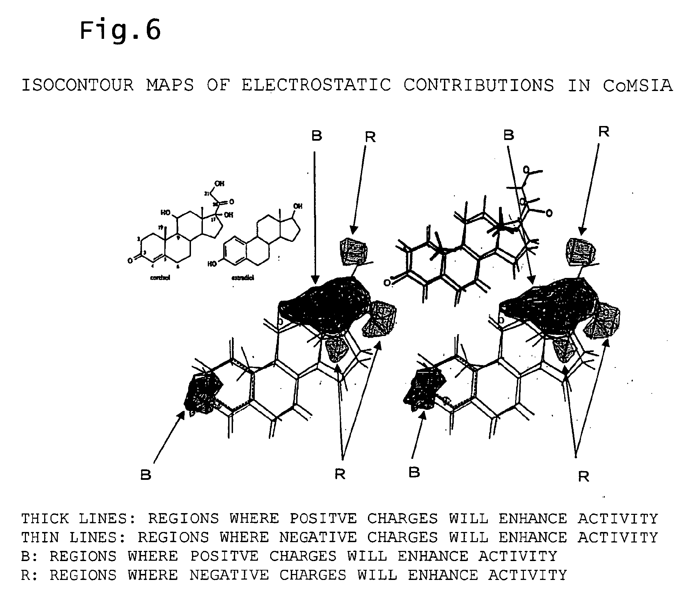

For comparison, in the example of CoMFA analysis according to SYBYL, Tripos Inc. St. Louis, 3D QSAR analysis was performed only on steric factors of a substitutional group influencing the activity, showing a result that the 17-position and the following side chains had regions where the activity would be enhanced sterically and regions where the activity would be suppressed and that activity-suppressing regions appeared around the 3-position of the A-ring. Meanwhile, the reported result of QSAR analysis with application of the three parameters of a steric term, an electrostatic term and a hydrophobic term to CoMSIA, as shown in Fig. 5, is almost the same as that of CoMFA, except that as for the steric contribution, only regions (in green: G) where the activity would be enhanced sterically appeared at the 17-position and the following side chains. As shown in Fig. 6, the electrostatic contribution as well appeared at the 17-position and the following side chains, suggesting that negatively charged oxygen atoms play a role in enhancement of the activity particularly at the 17-position side chain.

### III. COMPUTING IN THE PRESENT INVENTION

The following is the results of 3D QSAR calculation according to the present invention.

### III-1. COMPUTING OF STERIC INTERACTIONS AND ELECTROSTATIC INTERACTIONS

As a way of setting a represented point, two approaches were tried, one demanding generation of a represented point through superposition based only on atomic coordinates (Example 1) and another according to which pseudo-atoms were inserted at the center of rings and superposed with atomic coordinates and represented points were generated as positions representing the rings (Example 2).

### (1) EVALUATION FUNCTION

The four formulae and the like were used as evaluation functions.

### A) RAPID MOLECULAR SUPERPOSITION EVALUATION FORMULA

(Kotani, T.; Higashiura, K. Rapid evaluation of molecular shape similarity index using pairwise calculation of the nearest atomic distances. J. Chem. Inf. Comput. Sci., 2002, 42, 58-63.)

### B) SEAL-TYPE EVALUATION FORMULA

(Kearsley, S. K.; Smith, G. M. An alternative method for the alignment of molecular structures: maximizing electrostatic and steric overlap. Tetrahedron Compt. Method. 1990, 3, 615-633, Klebe, G.; Mietzner, T.; Weber, F. Different approaches toward an automatic structural alignment of drug molecules: applications to sterol mimics, thrombin and thermolysin inhibitors. J. Comput. Aided Mol. Des. 1994, 8, 751-778.)

### C) MOLECULAR SIMILARITY EVALUATION FORMULA ACCORDING TO GOOD ET AL.

(Good, A. C.; Hodgkin, E. E.; Richatds, W. G. Utilization of Gaussian functions for the rapid evaluation of molecular similarity. J. Chem. Inf. Comput. Sci., 1992, 32, 188-191.)

### D) USE OF INDICATOR VARIABLES

(An indicator variable indicative of the steric contribution is 1 when the position of the nearest atom to a represented point is equal to or smaller than a threshold value, 0.5 when the position of the nearest atom to the represented point is equal to or smaller than double the threshold value, and 0 when the position of the nearest atom to the represented point is not equal to or smaller than double the threshold value. An indicator variable indicative of the electrostatic contribution is the charge of the nearest atom when the position of the nearest atom to a represented point is equal to or smaller than the threshold value, half the charge of the nearest atom when the position of the nearest atom to the represented point is equal to or smaller than double the threshold value, and 0 when the position of the nearest atom to the represented point is not equal to or smaller than double the threshold value.)

Of these four evaluation formulae, the methods A) through C) are evaluation functions which are used to compute molecular similarity. Where the method A) is used as an evaluation function, although only the steric term is available in 3D QSAR analysis, it is possible to compute interactions between each represented point and each molecule at a high speed. By means of the evaluation functions according to the methods B) and C), as for those on which parameters have been reported, 3D QSAR is possible considering not only the steric contribution but the electrostatic contribution, hydrophobic interactions and the like as well. The method D) is an improved version of the method A, with which 3D QSAR is possible while taking into account electrostatic interactions. When such parameters as hydrophobic interactions, hydrogen donors and hydrogen acceptors are added, it is possible to compute interactions with these.

### (2) GENERATION OF REPRESENTED POINTS

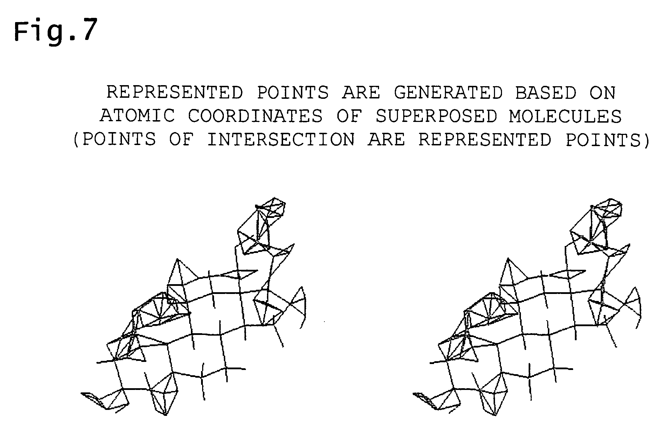

With respect to generation of a represented point, the threshold value for represented point generation through cluster analysis was set to 0.75 angstrom where a represented point was generated based only on atomic coordinates (Example 1). As represented points, 92 points were obtained (See Fig. 7.).

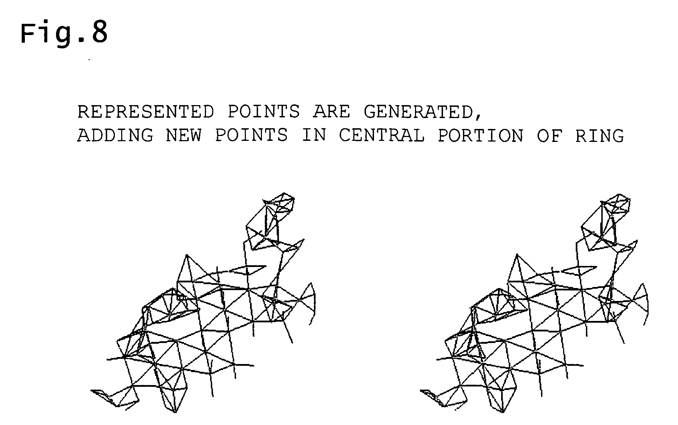

When a pseudo-atom is to be inserted at a position representing a ring, for molecules having a ring structure, a new atom (pseudo atom = point) may be added in a central section of the ring or the like to thereby generate a represented point. With this, the number of the represented points rose to 97 (See Fig. 8.). Since this increases the number of the represented points which consider characteristics of the ring portion of the molecules, the computing accuracy enhances.

Further, represented points obtained through cluster analysis are far less than thousands lattice points demanded in CoMFA, CoMSIA, etc. This not only shortens the computing time but reduces use of a memory area of a PC.

In CoMFA, scaling is not necessary as interactions at computed lattice points are all potential energies (kcal/mol). However, in CoMSIA and the present invention, descriptors of different units such as logP than potential energies are used, and therefore, scaling is needed for summation of the influences of the respective terms such as the hydrophobic term and the electrostatic terms. Noting this, in this approach, block-scaling was conducted.

### (3) 3D QSAR ANALYSIS

### Example 1 GENERATION OF REPRESENTED POINTS BASED ON ATOMIC COORDINATES OF SUPERPOSED MOLECULES

### 1-A) USE OF RAPID MOLECULAR SUPERPOSITION EVALUATION FORMULA

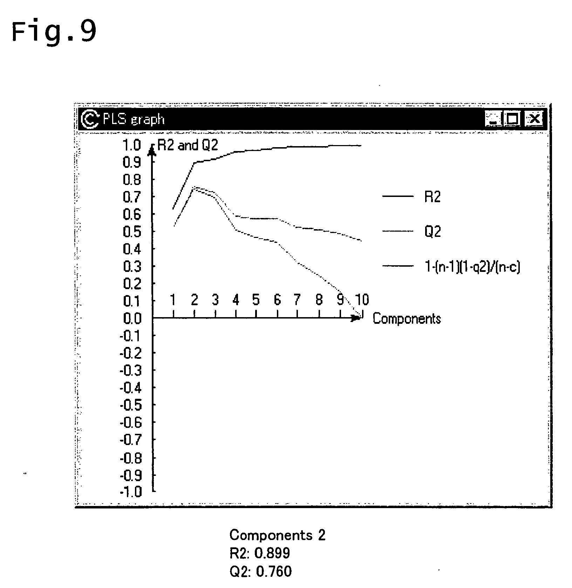

After superposition, 3D QSAR analysis using molecular superposition method discussed in Kotani, T.; Higashiura, K. Rapid evaluation of molecular shape similarity index using pairwise calculation of the nearest atomic distances. J. Chem. Inf. Comput. Sci., 2002, 42, 58-63. was conducted. Fig. 9 shows the PLS analysis result. In Fig. 9, r² is a multiple correlation coefficient, q² is cross-validated r², and 1-(n-1)(1- q²)/(n-c) is an evaluation function expressing the optimal number of components proposed by Tropsha et al. In this example, q² has the maximum value when the number of components is 2, holding that this is a reliable model.

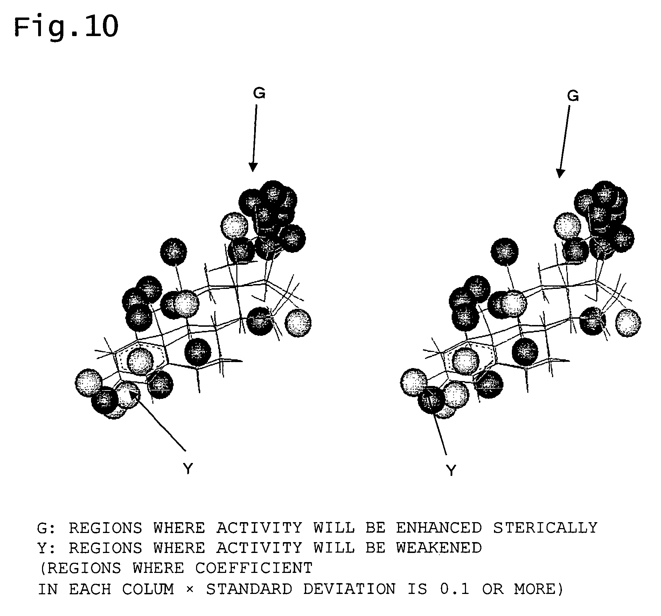

Fig. 10 is visualization of the computed result. In Fig. 10, the green portions are regions where the activity will be enhanced sterically, i.e., a sterically demanding substitutional group will enhance the activity, while the yellow portions are the opposite regions, namely, regions where the sterically demanding substitutional group will weaken the activity. This result is in approximate agreement with CoMFA, CoMSIA, etc. However, a region unfound in CoMFA, CoMSIA and the like exists near the 15-position of the D-ring.

### 1-B) USE OF SEAL-TYPE EVALUATION FORMULA

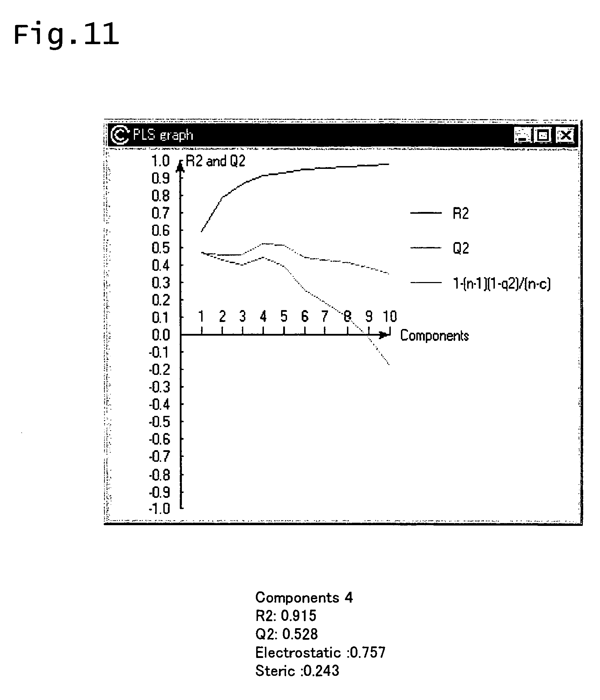

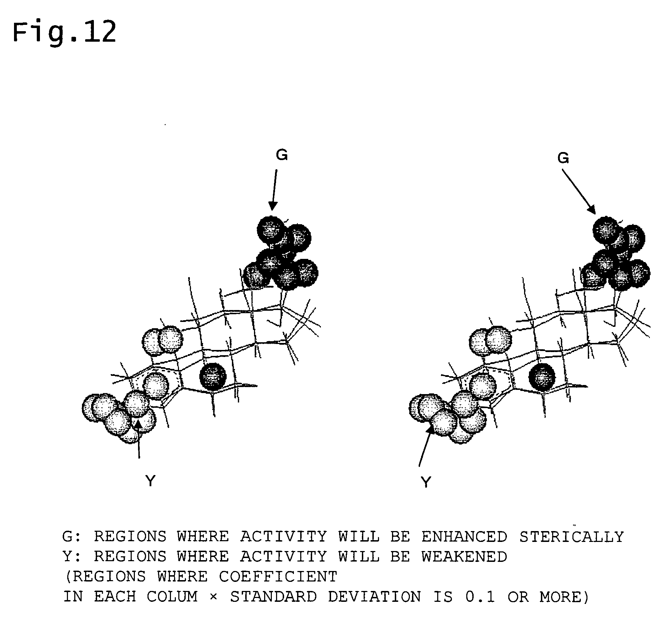

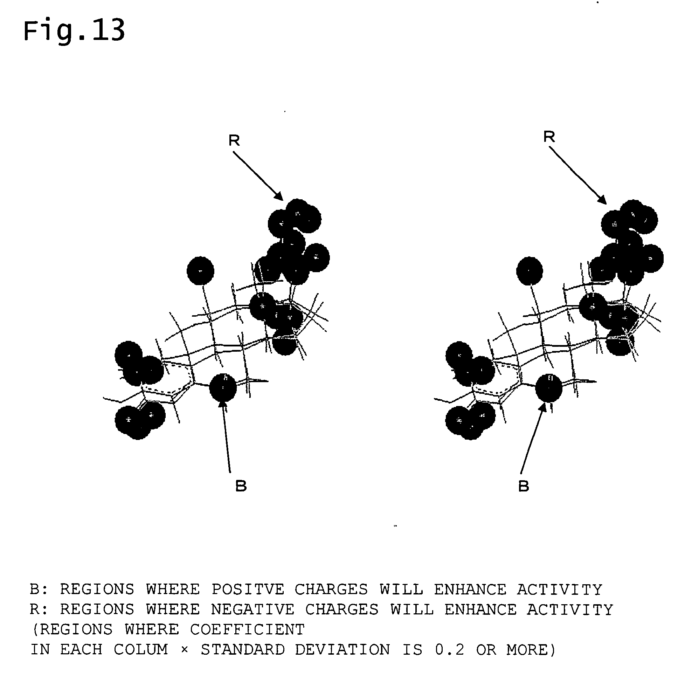

3D QSAR analysis then followed using a SEAL-type evaluation formula. Fig. 11 is a graph of r², q² and 1-(n-1) (1- q²)/(n-c). In Fig. 11, q² has the maximum value when the number of components is 4, indicating the highest reliability of analysis is attained under this condition. In this example, it was possible to evaluate the electrostatic term as well, not just the steric term. Figs. 12 and 13 respective show them. As shown in the drawings, the results are similar to the CoMSIA results as for the steric and the electrostatic contributions.

### 1-C) USE OF MOLECULAR SIMILARITY EVALUATION FORMULA ACCORDING TO GOOD ET AL.

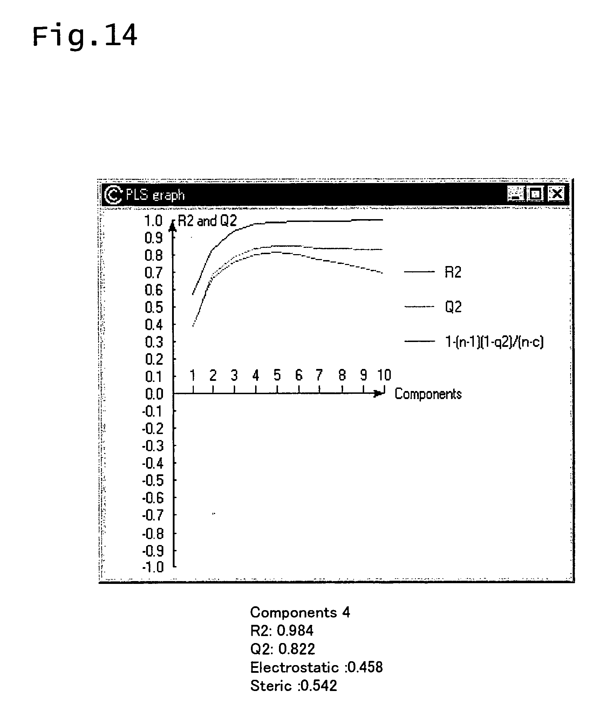

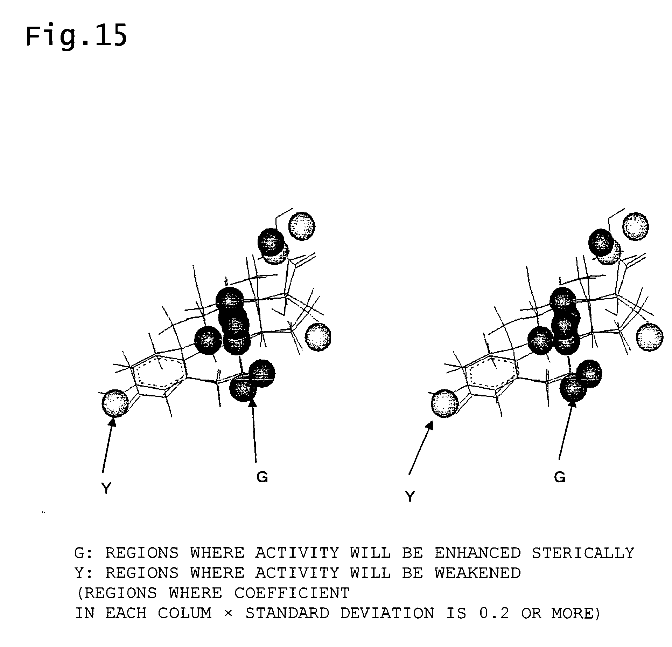

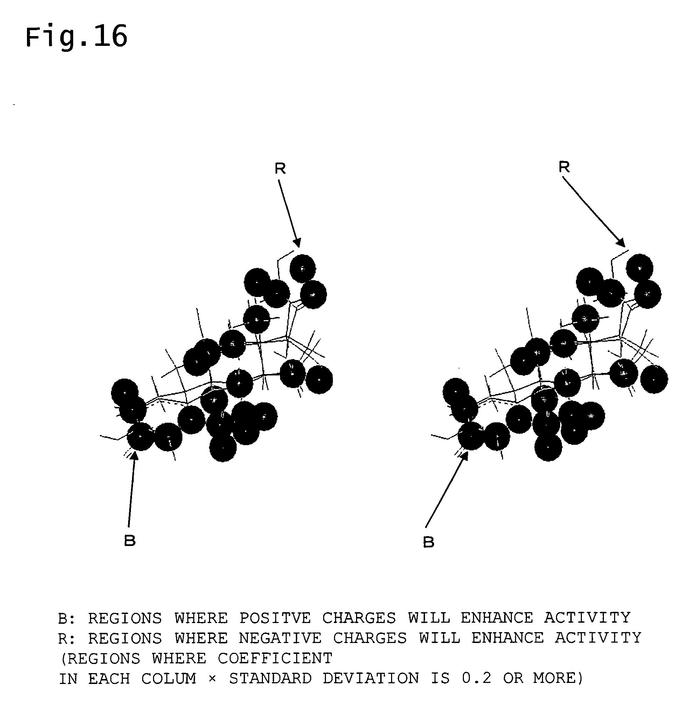

Fig. 14 is a graph of r², q² and 1-(n-1)(1-q²)/(n-c) as they are when the Good's evaluation formula on molecular similarity is used. In Fig. 14, when the number of components is 4, q² is as high as 0.822. This means that this model is extremely reliable. However, the drawings (Figs. 15 and 16) illustrating the contributions of the steric and the electrostatic terms are considerably different from those which represent the above three instances.

### 1-D) USE OF INDICATOR VARIABLES

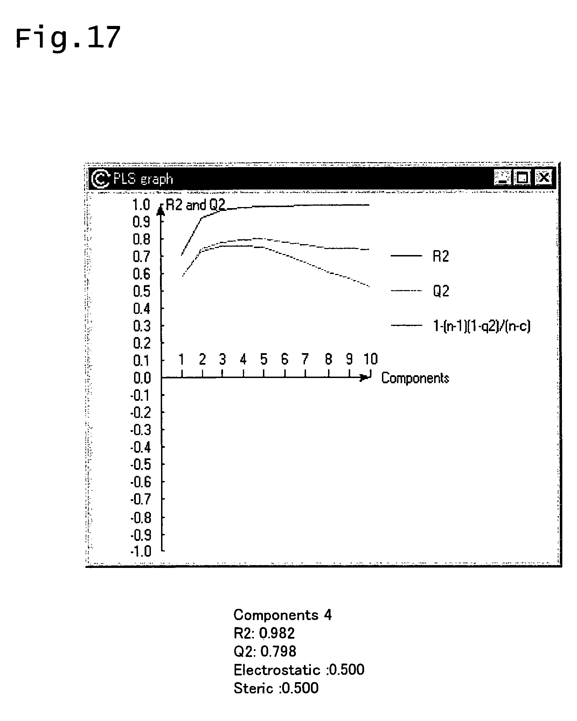

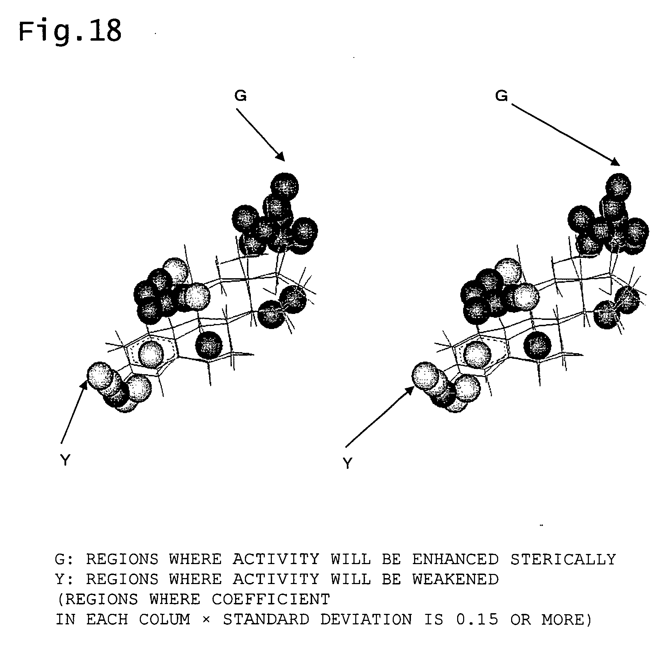

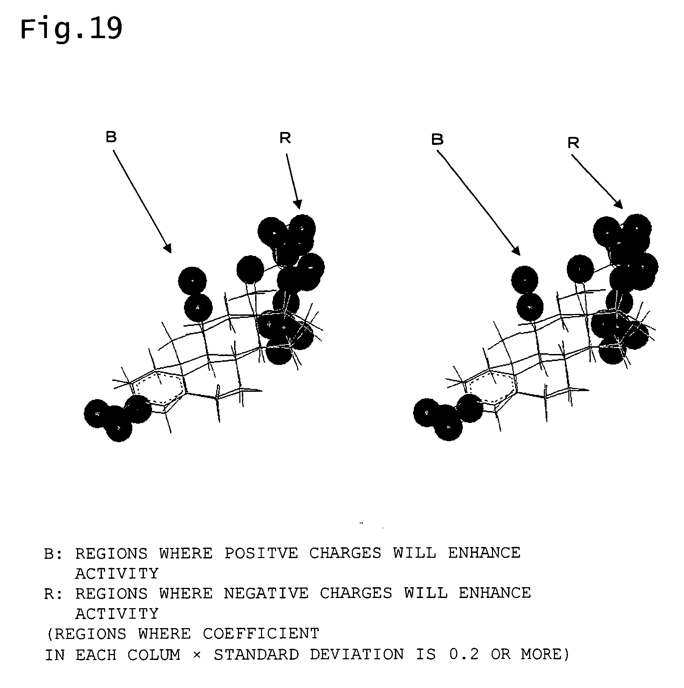

Fig. 17 is a graph of r², q² and 1-(n-1)(1-q²)/(n-c) as they are when as indicator variables, the steric and the electrostatic factors are both set to 0.5. When the number of components is 4, q² is the maximum. Figs. 18 and 19 show 3D QSAR analysis results under this condition. In this example, the drawings which show the contribution of the steric term are similar to the CoMFA and CoMSIA results, and the drawings which show the contribution of the electrostatic term are similar to the CoMSIA result. The result regarding the contribution of the steric term is similar to the result obtained from the 1-A) rapid molecular superposition evaluation formula.

### Example 2 GENERATION OF REPRESENTED POINT WITH ADDITION OF NEW POINT AT POSITION REPRESENTING RING

A new point (pseudo-atom) wad added in a central section of a ring as a position which represents the ring, and similar computing was conducted. Addition of the pseudo-atom is expected to improve the accuracy of superposition and yield a more precise 3D QSAR result.

### 2-A) USE OF RAPID MOLECULAR SUPERPOSITION EVALUATION FORMULA

The same result as the 1-A result was obtained. This means that the rapid molecular superposition method which the inventors have developed is so accurate that it is not necessary to insert a pseudo-atom and permits superposition of molecules at a high accuracy.

### 2-B) USE OF SEAL-TYPE EVALUATION FORMULA

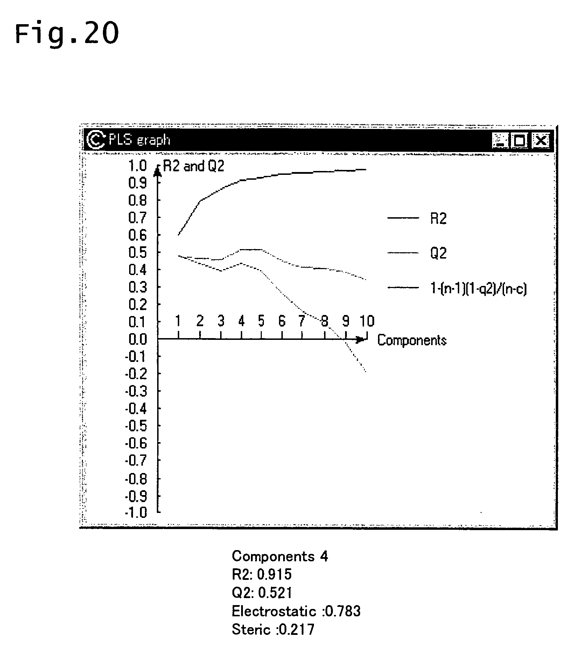

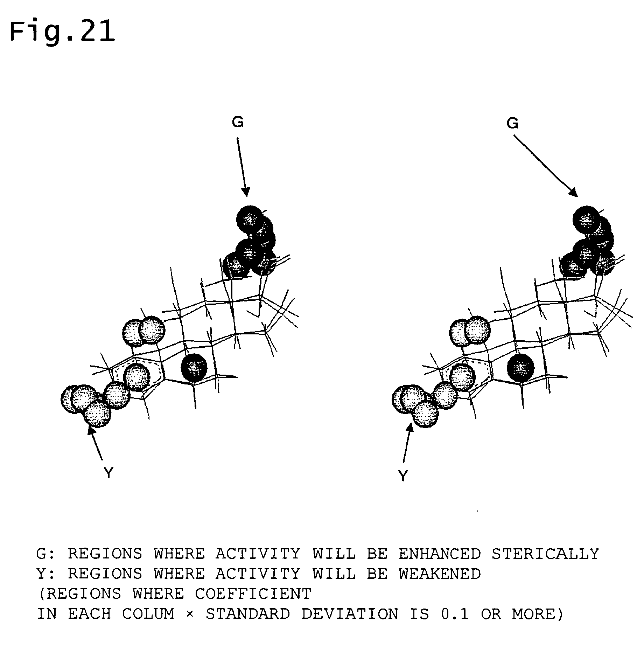

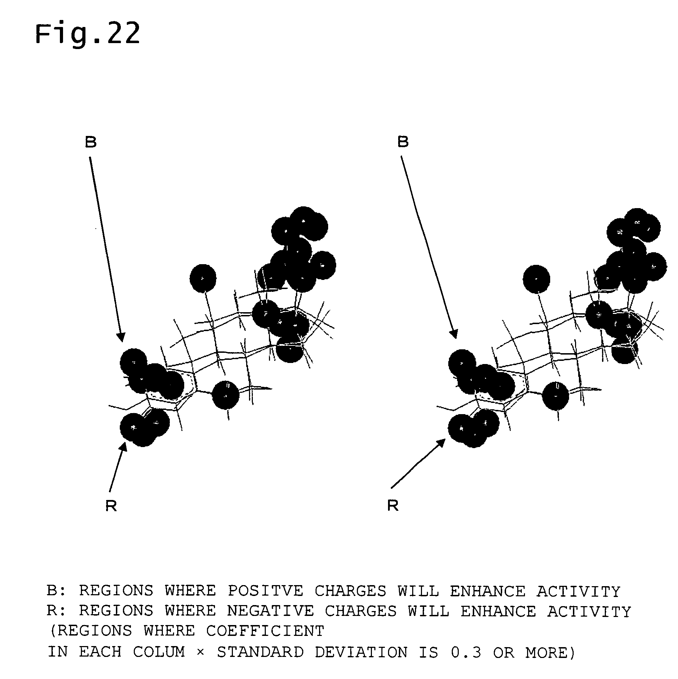

After superposition with a pseudo-atom inserted at the center of a ring, 3D QSAR analysis was conducted using the SEAL-type evaluation method. Fig. 20 is a graph of r², q² and 1-(n-1)(1- q²)/(n-c). In this case, as q² has the maximum value when the number of components is 4, analysis under this condition is found most reliable. Figs. 21 and 22 are drawings of the contributions of the steric and the electrostatic terms. As compared with the situation (Example 1-B) that a pseudo-atom is not inserted at the center of a ring, the result on the electrostatic term is exactly the same and the result on the steric term is almost the same.

### 2-C) USE OF MOLECULAR SIMILARITY EVALUATION FORMULA ACCORDING TO GOOD ET AL.

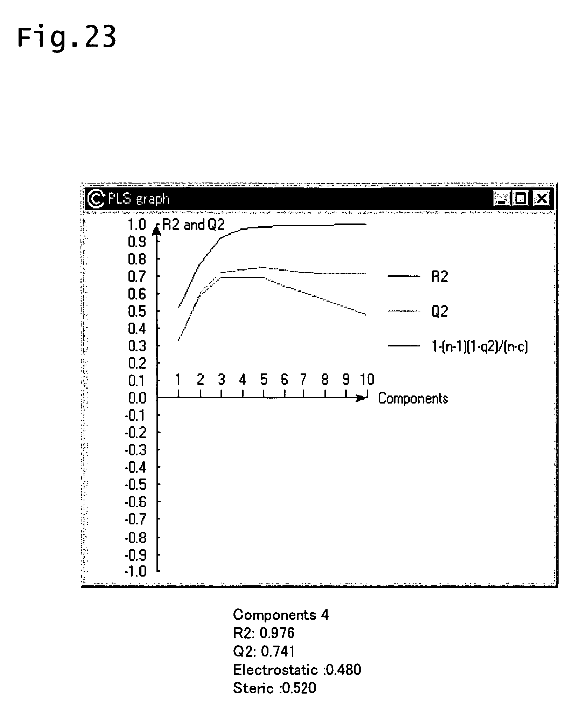

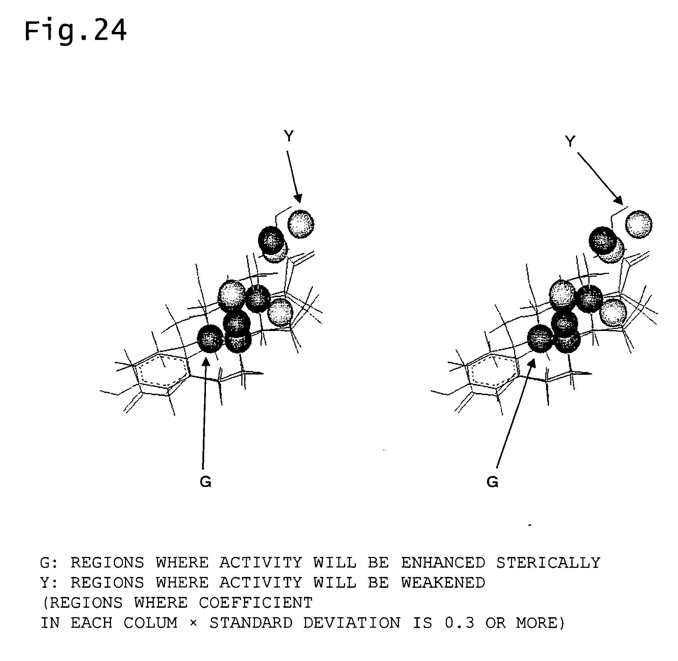

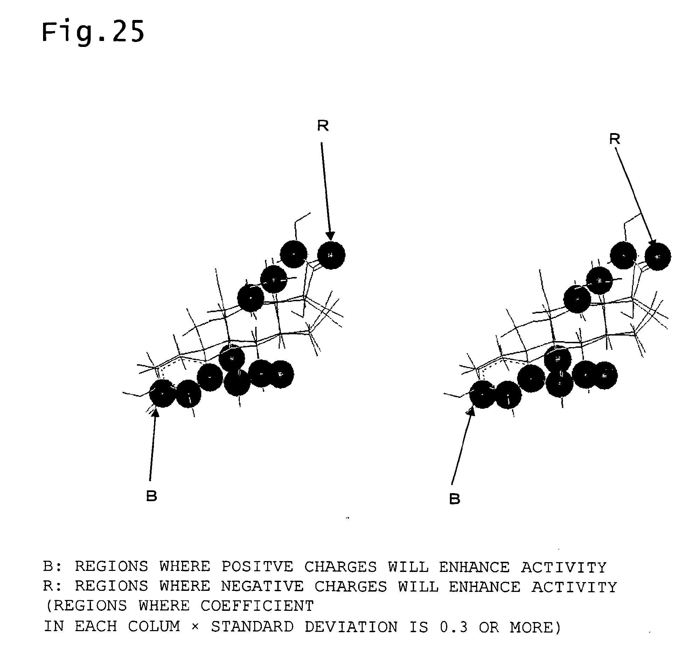

Fig. 23 is a graph of rr², q² and 1-(n-1)(1- q²)/(n-c) as they are when the Good's evaluation formula on molecular similarity is used. In Fig. 23, when the number of components is 4, q² is as high as 0.741. Although this means that this model is extremely reliable, the drawings (Figs. 24 and 25) which show the contributions of the steric and the electrostatic terms are remarkably different from the CoMSIA and the other results above as in the case of the example 1-C.

### 2-D) USE OF INDICATOR VARIABLES

The same result as the 1-D result was obtained.

Analyzing the method according to the present invention in light of the evaluation formulae which were used, it is found that although 3D QSAR analysis resulted in higher values of both r² and q² than those yielded from the other evaluation functions in the situations (1-C, 2-C) that the Good's evaluation formulae on molecular similarity were used, the activity-affecting regions were remarkably different from the CoMFA and CoMSIA results, suggesting the need to further study the method as a 3D QSAR evaluation function. Use of the SEAL-type evaluation method (1-B, 2-B), although being the same Gaussian-type evaluation method, created an approximately similar result to the isocontour maps representing CoMFA (steric contribution), CoMSIA, etc., but produced slightly lower r² and q² than those yielded from the other evaluation functions. On the contrary, when the rapid molecular superposition evaluation method was used (1-A) and the indicator variables were used (1-D), r² and q² were both higher than the CoMFA and CoMSIA results. As compared with where the SEAL-type evaluation function was used, the activity-affecting regions were approximately similar although including a region having somewhat different property. As for determination of a represented point through cluster analysis, the 3D QSAR results were not greatly different between where the new points were added in the central sections of the rings as positions representing the rings and where new points were not added in the central sections of the rings as positions representing the rings.

### III-2. COMPUTING OF HYDROPHOBIC INTERACTIONS

Among various methods to compute a hydrophobic contribution, AlogP according to Viswanadhan et al., the evaluation function SEAL method used in CoMSIA, i.e., a procedure of computing hydrophobic interactions (Viswanadhan, V. N.; Ghose, A. K.; Singh, U. C.; Wendoloski, J. J. Prediction of Solvation Free Energies of Small Organic Molecules: Additive-Constitutive Models Based on Molecular Fingerprints and Atomic Constants. J. Chem. Inf. Comput. Sci., 1999, 39, 405-412), and hydrophobic interaction parameters used in FLEXS, which is a rapid superposition method considering the degree of freedom developed by Klebe et al., were used as hydrophobic interaction evaluation functions in the present invention. In addition, parameters used in HASL were applied to the present invention, although these were not parameters indicative only of hydrophobic interactions.

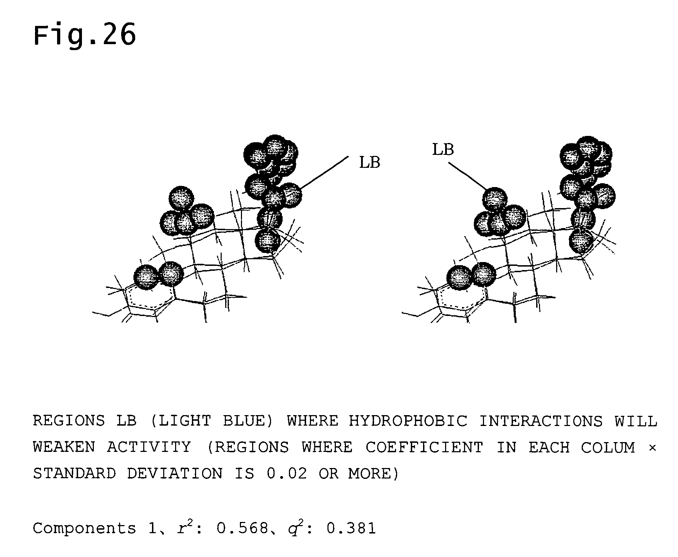

Each parameter was examined using a Gaussian-type function as in the case of SEAL and two types of functions to which indicator variables were applied. A similar procedure to III-1 was followed for generation of represented points and 3D QSAR analysis. Represented points were generated without adding a pseudo-atom at the center of a ring. Fig. 26 shows the computed result. In Fig. 26, the orange portions are regions where hydrophobic interactions will enhance the activity, while the light blue portions are regions where hydrophobic interactions will weaken the activity, that is, regions where hydrophilic interactions will enhance the activity.

As hydrophobic parameters, the following parameters were used.
3) hydrophobic parameter used in SEAL (AlogP according to Viswanadhan et al.)
4) hydrophobic parameter used in FLEXS
5) hydrophobic parameter used in HASL

Meanwhile, the two methods E and F below were used as evaluation functions.

### E) USE OF GAUSSIAN-TYPE EVALUATION FORMULA

While various attenuation curves expressing hydrophobic interactions have been reported, the formula below was used as a Gaussian-type evaluation formula for CoMSIA. 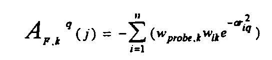 where A_{F,k} denotes an interaction between a molecule j and a represented point q. The symbol Wᵢₖ denotes a value assigned to each physiochemical property of an atom i, while the symbol W_{probe,k} denotes a value assigned to each physiochemical property of a probe atom. As hydrophobic parameters, parameter values in SEAL, FLEXS or HASL were applied. The probe atom had a charge of 1, the atomic radius of 1 angstrom and the hydrophobicity of 1. The symbol α is a coefficient of an index and the symbol r_{iq} denotes the distance between the probe atom on the represented point and the point i on a molecule at which the interaction is to be calculated. In the present invention, α is 0.3.

### F) USE OF INDICATOR VARIABLES

An indicator variable indicative of the hydrophobic contribution is, when the position of the nearest atom to a represented point is equal to or smaller than a threshold value, a parameter value dependent upon the atom type, but is a value obtained by multiplying the parameter by 0.5 when the position is equal to or smaller than double the threshold value and is 0 when the position is not equal to or smaller than double the threshold value.

The hydrophobic contribution according to the present invention was evaluated, with the total six procedures combining the hydrophobic parameters and the evaluation functions above.

### 3-E) USE OF GAUSSIAN-TYPE EVALUATION FORMULA WITH APPLICATION OF SEAL HYDROPHOBIC PARAMETER

3D QSAR analysis was conducted applying the parameter for SEAL to the Gaussian-type evaluation formula. (Fig. 26) 3-F) USE OF INDICATOR VARIABLES WITH APPLICATION OF SEAL HYDROPHOBIC PARAMETER

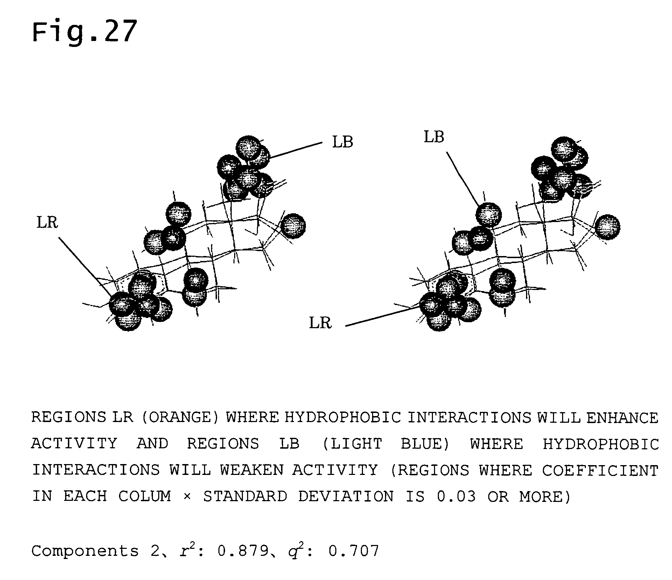

3D QSAR analysis was conducted applying the parameter for SEAL to indicator variables. (Fig. 27)

### 4-E) USE OF GAUSSIAN-TYPE EVALUATION FORMULA WITH APPLICATION OF FLEXS HYDROPHOBIC PARAMETER

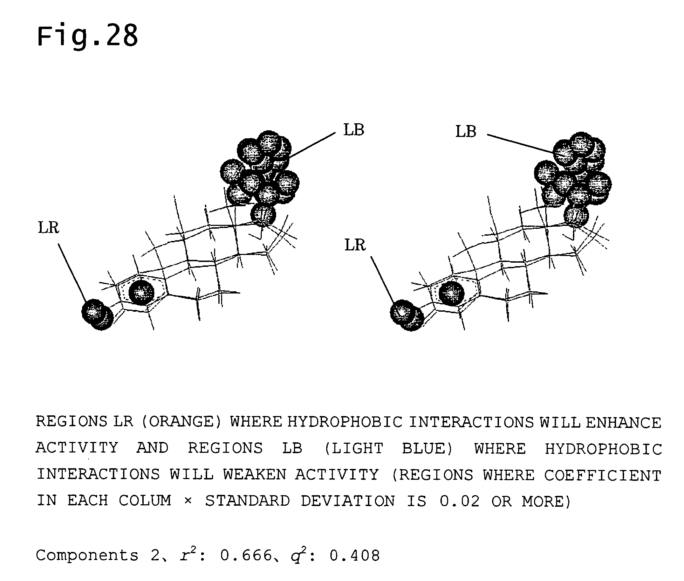

3D QSAR analysis was conducted applying the hydrophobic parameter for FLEXS to the Gaussian-type evaluation formula. (Fig. 28)

### 4-F) USE OF INDICATOR VARIABLES WITH APPLICATION OF FLEXS HYDROPHOBIC PARAMETER

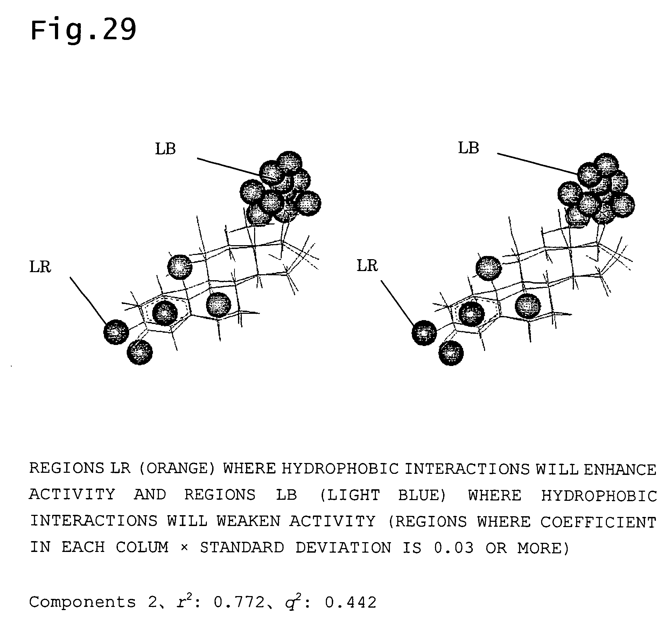

3D QSAR analysis was conducted applying the hydrophobic parameter for FLEXS to indicator variables. (Fig. 29)

### 5-E) USE OF GAUSSIAN-TYPE EVALUATION FORMULA WITH APPLICATION OF HASL PARAMETER

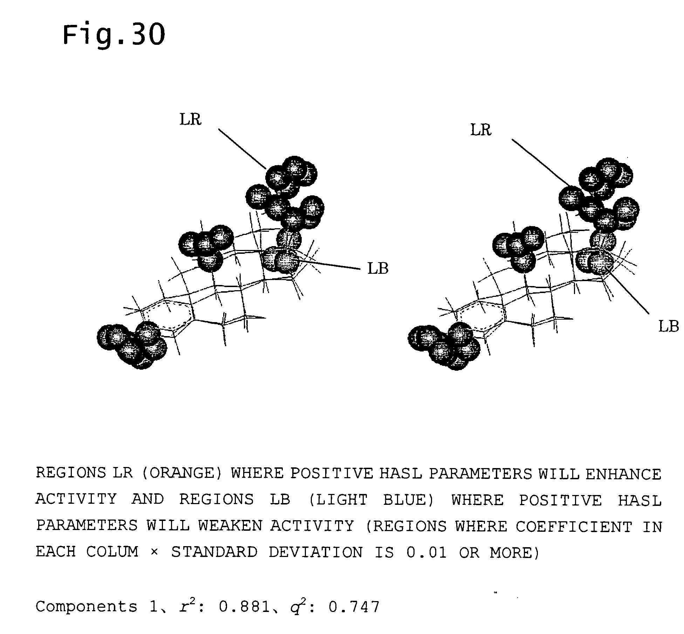

3D QSAR analysis was conducted applying the parameter for HASL to the Gaussian-type evaluation formula. (Fig. 30) 5-F) USE OF INDICATOR VARIABLES WITH APPLICATION OF HASL PARAMETER

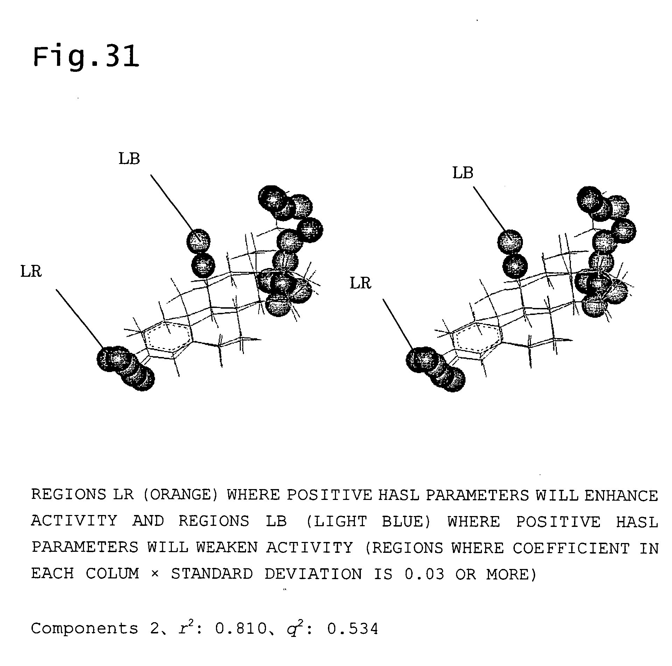

3D QSAR analysis was conducted applying the parameter for HASL to indicator variables. (Fig. 31)

### III-3. INFLUENCES OF ATTENUATION FUNCTIONS USED IN CALCULATION OF INTERACTIONS

The accuracy of 3D QSAR has been discussed in the present invention, using the two types of Gaussian-type attenuation functions and indicator variables. It is indicated that the application of molecular similarity developed by Good et al. to the present invention, although producing high r² and q² than where other approaches are used, results in a very different activity-affecting region from the CoMFA, CoMSIA and other results, and as such is not appropriate for 3D QSAR. It is clear that when the SEAL-type evaluation formula is used, although this involves use of the same Gaussian function in calculation of interactions expressing physiochemical properties, an approximately the same result is obtained as the isocontour maps representing CoMFA (steric contribution), CoMSIA, etc.

Noting this, consideration was given on influences of the application of the three types of attenuation functions (Formulae 1 through 3) for MLP upon the accuracy of the method according to the present invention. As molecular lipophilic potential (MLP) potentials introduced to CoMFA analysis for calculation of hydrophobic interactions, besides the attenuation functions used in CoMSIA, CoMFA, etc., attenuation functions such as the Audry formula (FORMULA 1) (Furet, P.; Cohen, N. C. 3D molecular lipophilicity potential profiles: a new tool in molecular modeling. J. Mol. Graph. 1988, 6, 182-189), the Fauchère formula (FORMULA 2) (Fauchère, J. L.; Quarendon, P.; Kaetterer, L. Estimating and representing hydrophobicity potential. J. Mol. Graph. 1988, 6, 202-206) and the modified Fauchère formula (FORMULA 3) (Kearsley, S. K.; Smith, G. M. An alternative method for the alignment of molecular structures: maximizing electrostatic and steric overlap. Tetrahedron Compt. Method. 1990, 3, 615-633) have been proposed.

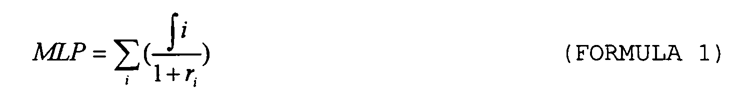

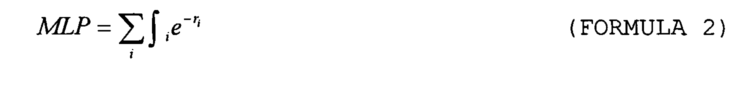

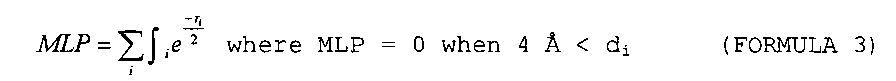

The symbol fᵢ denotes the hydrophobic constant of an i-th atom (fragment). These attenuation functions are designed so that the value at the atom (fragment) is 1 and the distance is zero at the infinite limit.

The four types (6), (7), (8) and (9) below were studied as physiochemical properties.
(6) STERIC INTERACTIONS
(7) ELECTROSTATIC INTERACTIONS
(8) HYDROPHOBIC INTERACTIONS USING FLEXS PARAMETER
(9) HYDROPHOBIC INTERACTIONS USING AlogP PARAMETER

As attenuation functions, the formulae 1 through 3, the SEAL-type Gaussian function and indicator variables were used.

### (G) USE OF AUDRY FORMULA (FORMULA 1) AS ATTENUATION FUNCTION

### (H) USE OF FAUCHÈRE FORMULA (FORMULA 2) AS ATTENUATION FUNCTION

### (K) USE OF MODIFIED FAUCHÈRE FORMULA (FORMULA 3) AS ATTENUATION FUNCTION

To compare against the attenuation function, the following was used.

### (J) SEAL-TYPE GAUSSIAN FUNCTION

### (K) INDICATOR VARIABLES USED IN SECTION F OF THE INVENTION

On each attenuation function, influences over the steric interactions (6), the electrostatic interactions (7) and the hydrophobic interactions (8) and (9) were computed. The probe atom had a charge of 1 and the atomic radius of 1 angstrom.

A similar procedure to III-1 and III-2 was followed for generation of represented points and 3D QSAR analysis. Represented points were generated without adding a pseudo-atom at the center of a ring.

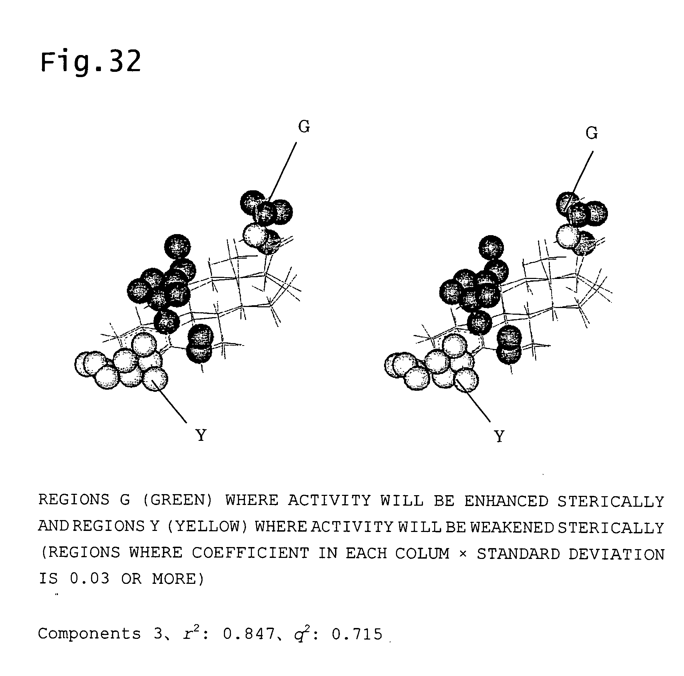

Fig. 32 and the subsequent drawings show the computed results. In these drawings, the same color chart to those used in III-1 and III-2 is used for the respective regions.

Of the combinations between the physiochemical parameters (6) through (9) and the attenuation functions (G) through (K) above, the following 17 combinations were subjected to 3D QSAR analysis. For evaluation of the 3D QSAR analysis results, a multiple correlation coefficient (r²) and cross-validated r² (q²) were used.

### (6) EXAMINATION OF STERIC INTERACTIONS

Using the five types of attenuation functions, the influences over the steric interactions were studied.

### 6-G) EXAMINATION OF STERIC INTERACTIONS USING AUDRY FORMULA (FORMULA 1) AS ATTENUATION FUNCTION

The steric interactions were studied using the Audry formula as an attenuation function. Fig. 32 shows the result.

### 6-H) EXAMINATION OF STERIC INTERACTIONS USING FAUCHÈRE FORMULA (FORMULA 2) AS ATTENUATION FUNCTION

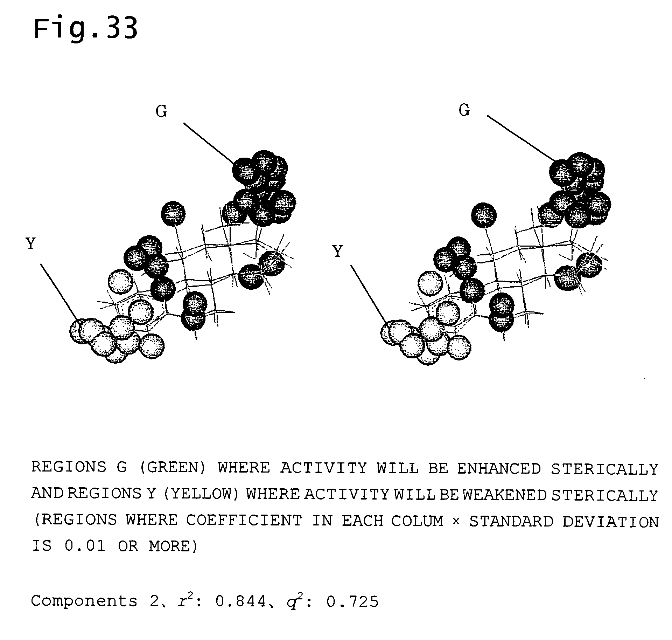

The steric interactions were studied using the Fauchère formula as an attenuation function. Fig. 33 shows the result.

### 6-I) EXAMINATION OF STERIC INTERACTIONS USING MODIFIED FAUCHÈRE FORMULA (FORMULA 3) AS ATTENUATION FUNCTION

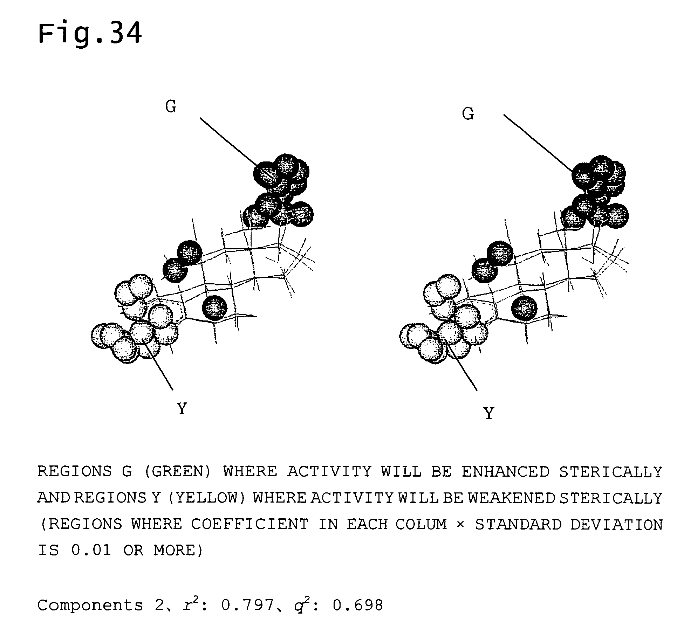

The steric interactions were studied using the modified Fauchère formula as an attenuation function. Fig. 34 shows the result.

### 6-J) EXAMINATION OF STERIC INTERACTIONS USING SEAL-TYPE GAUSSIAN FUNCTION

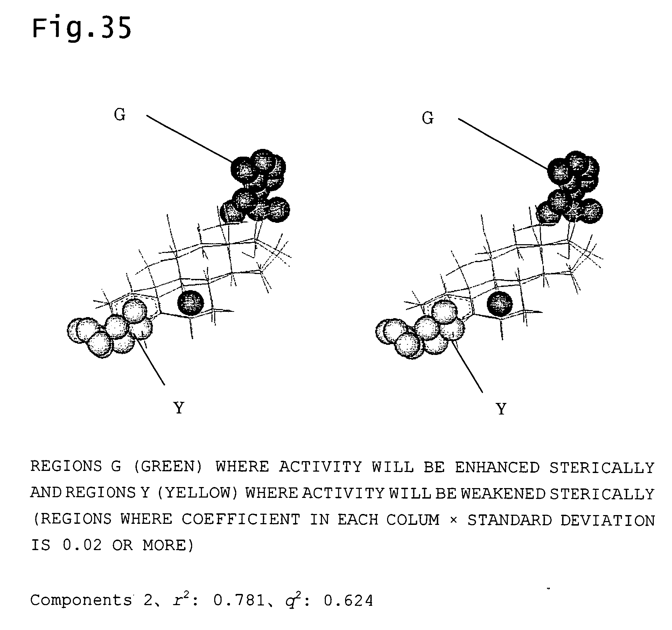

The steric interactions were studied using the SEAL-type Gaussian function. Fig. 35 shows the result.

### 6-K) EXAMINATION OF STERIC INTERACTIONS USING INDICATOR VARIABLES USED IN SECTION F OF THE INVENTION

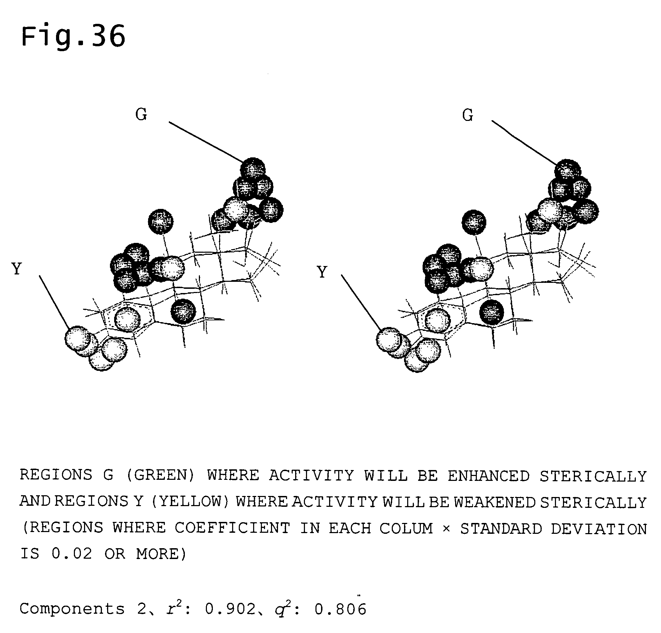

The steric interactions were studied using the indicator variables used in the section F of the present invention. Fig. 36 shows the result.

### (7) EXAMINATION OF ELECTROSTATIC INTERACTIONS

Using the five types of attenuation functions, the influences over the electrostatic interactions were studied.

### 7-G) EXAMINATION OF ELECTROSTATIC INTERACTIONS USING AUDRY FORMULA AS ATTENUATION FUNCTION

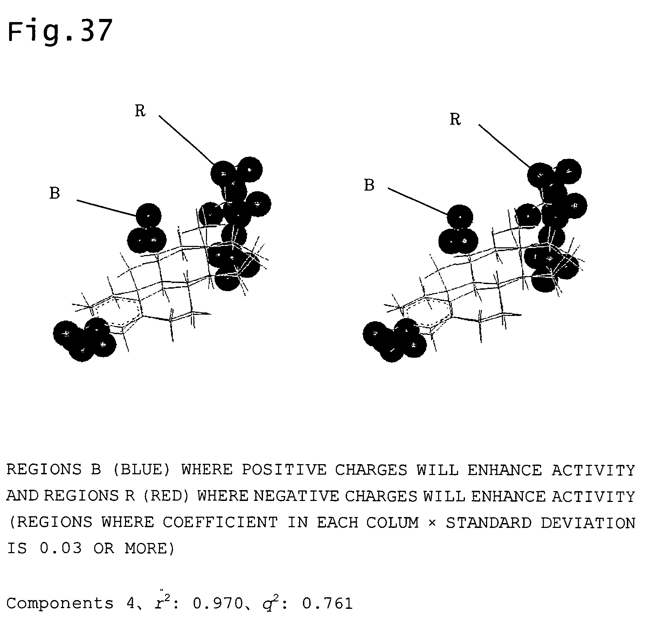

The electrostatic interactions were studied using the Audry formula as an attenuation function. Fig. 37 shows the result.

### 7-H) EXAMINATION OF ELECTROSTATIC INTERACTIONS USING FAUCHÈRE FORMULA AS ATTENUATION FUNCTION

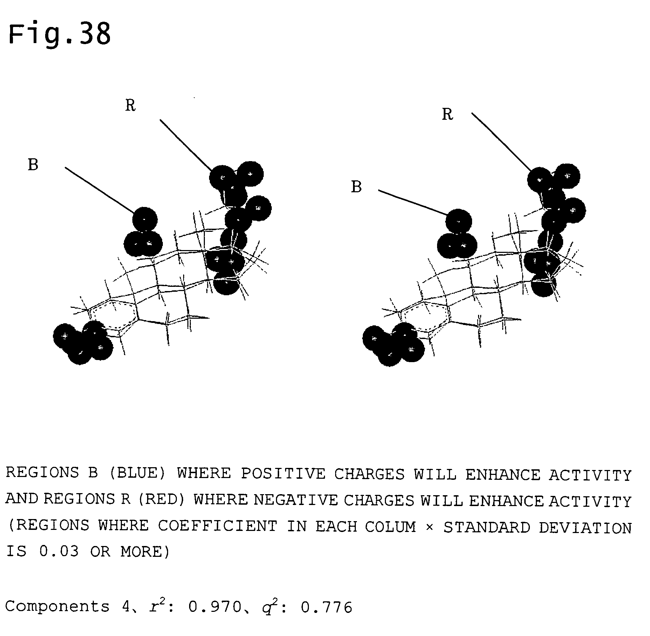

The electrostatic interactions were studied using the Fauchère formula as an attenuation function. Fig. 38 shows the result.

### 7-I) EXAMINATION OF ELECTROSTATIC INTERACTIONS USING MODIFIED FAUCHÈRE FORMULA AS ATTENUATION FUNCTION

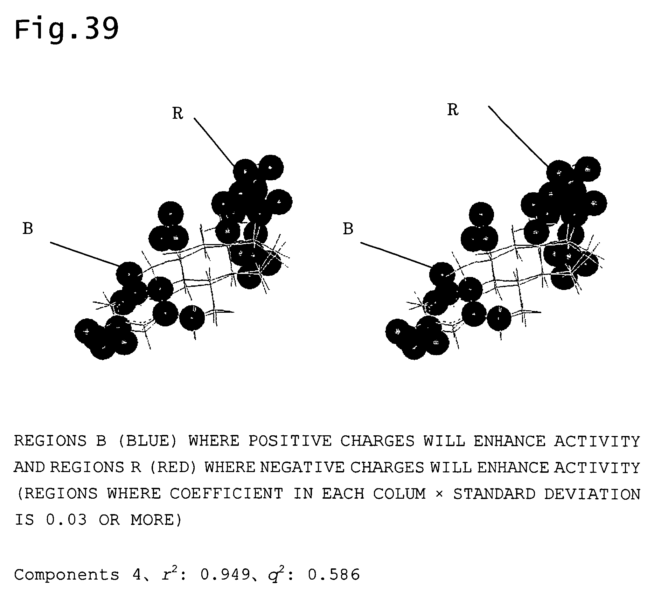

The electrostatic interactions were studied using the modified Fauchère formula as an attenuation function. Fig. 39 shows the result.

### 7-J) EXAMINATION OF ELECTROSTATIC INTERACTIONS USING SEAL-TYPE GAUSSIAN FUNCTION

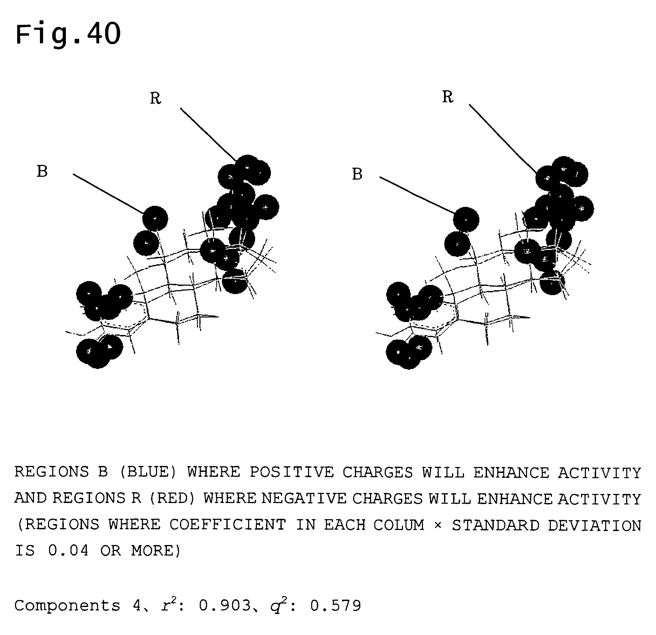

The electrostatic interactions were studied using the SEAL-type Gaussian function. Fig. 40 shows the result.

### 7-K) EXAMINATION OF ELECTROSTATIC INTERACTIONS USING INDICATOR VARIABLES USED IN SECTION F OF THE INVENTION

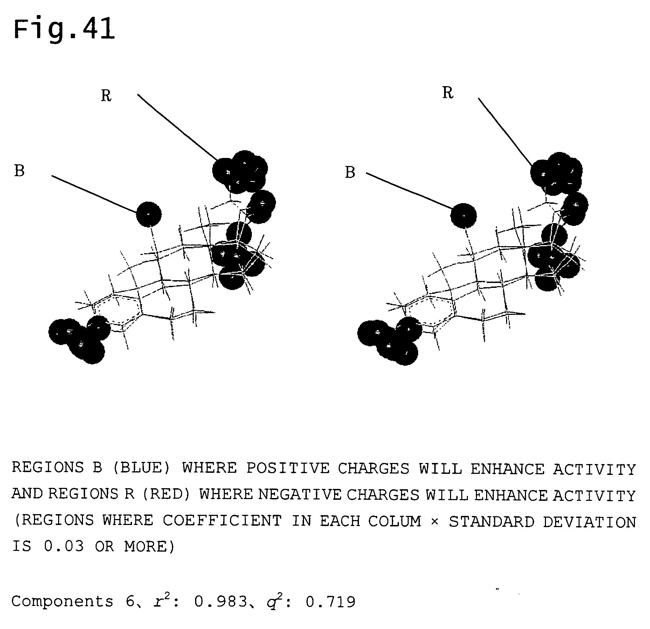

The electrostatic interactions were studied using the indicator variables used in the section F of the present invention. Fig. 41 shows the result.

### (8) EXAMINATION OF HYDROPHOBIC INTERACTIONS USING FLEXS PARAMETER

Since favorable results were not obtained using the indicator variables (K), excluding this, the four types of attenuation functions (G through J) were used to study the influences over 3D QSAR.

### 8-G) EXAMINATION OF HYDROPHOBIC INTERACTIONS USING AUDRY FORMULA AS ATTENUATION FUNCTION

The hydrophobic interactions were studied using the Audry formula as an attenuation function, failing to identify the optimal number of components.

### 8-H) EXAMINATION OF HYDROPHOBIC INTERACTIONS USING FAUCHÈRE FORMULA AS ATTENUATION FUNCTION

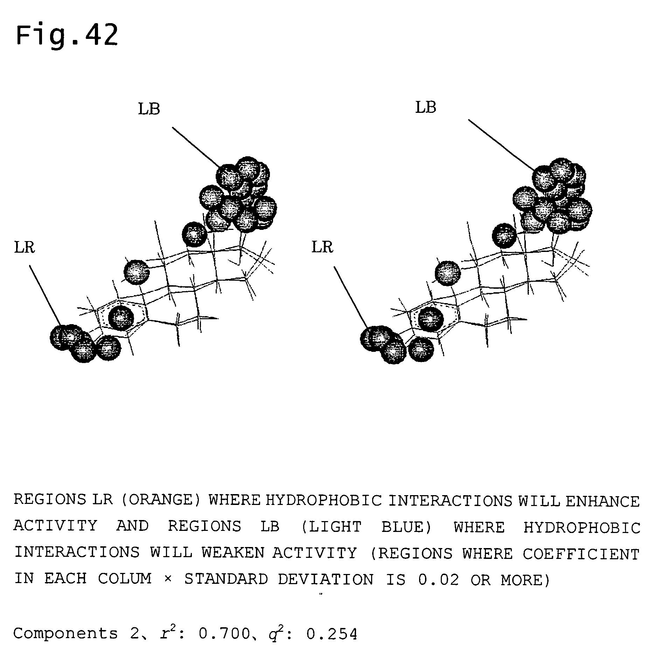

The hydrophobic interactions were studied using the Fauchère formula as an attenuation function. Fig. 42 shows the result.

### 8-I) EXAMINATION OF HYDROPHOBIC INTERACTIONS USING MODIFIED FAUCHÈRE FORMULA AS ATTENUATION FUNCTION

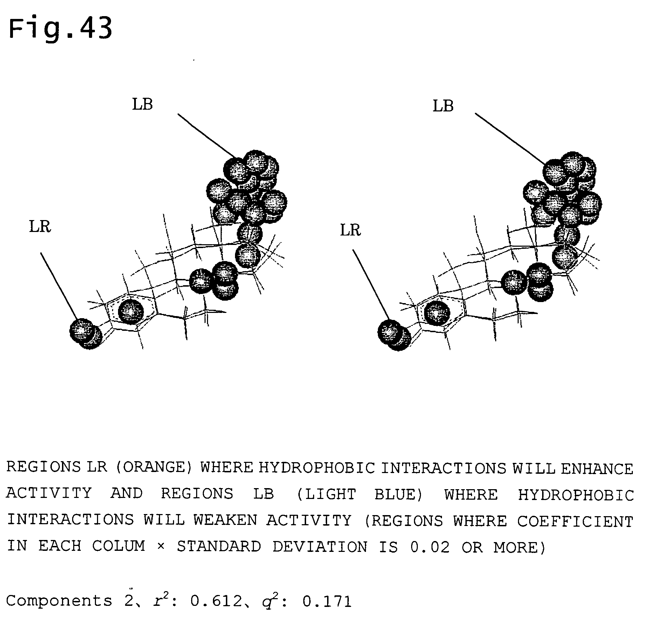

The hydrophobic interactions were studied using the modified Fauchère formula as an attenuation function. Fig. 43 shows the result.

### 8-J) EXAMINATION OF HYDROPHOBIC INTERACTIONS USING SEAL-TYPE GAUSSIAN FUNCTION

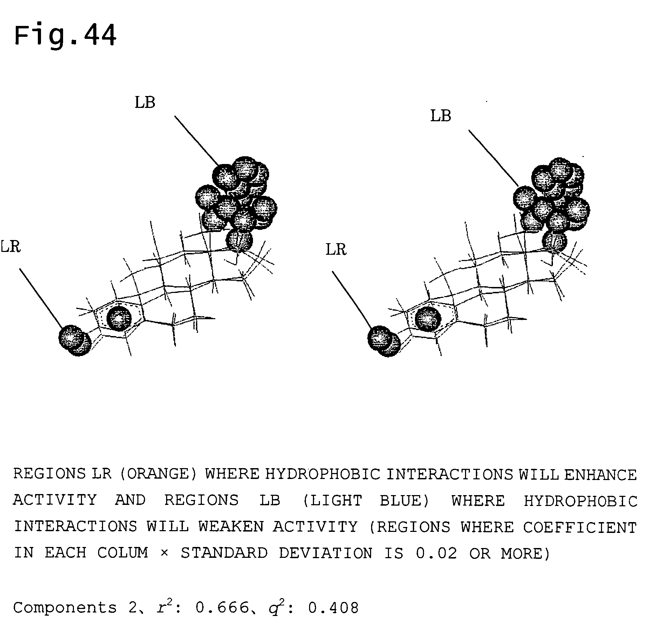

The hydrophobic interactions were studied using the SEAL-type Gaussian function. Fig. 44 shows the result.

### (9) EXAMINATION OF HYDROPHOBIC INTERACTIONS USING AlogP PARAMETER

Since favorable results were not obtained from the SEAL-type Gaussian function or the indicator variables while applying the AlogP parameter, excluding this, the three types of attenuation functions (G through I) were used to study the influences over 3D QSAR.

### 9-G) EXAMINATION OF HYDROPHOBIC INTERACTIONS USING AUDRY FORMULA AS ATTENUATION FUNCTION

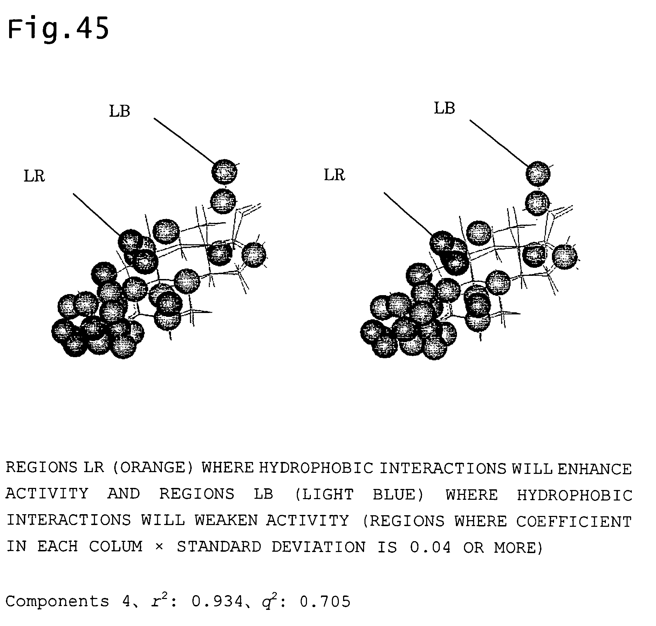

The hydrophobic interactions were studied using the Audry formula as an attenuation function. Fig. 45 shows the result.

### 9-H) EXAMINATION OF HYDROPHOBIC INTERACTIONS USING FAUCHÈRE FORMULA AS ATTENUATION FUNCTION

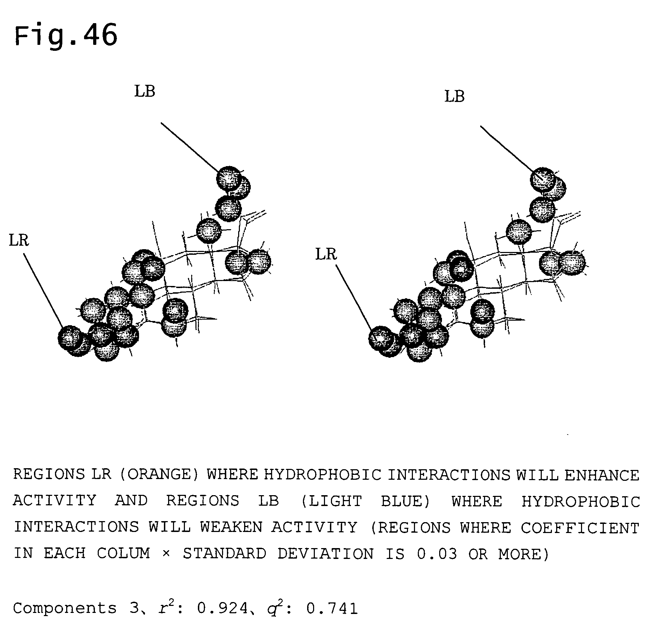

The hydrophobic interactions were studied using the Fauchère formula as an attenuation function. Fig. 46 shows the result.

### 9-I) EXAMINATION OF HYDROPHOBIC INTERACTIONS USING MODIFIED FAUCHÈRE FORMULA AS ATTENUATION FUNCTION

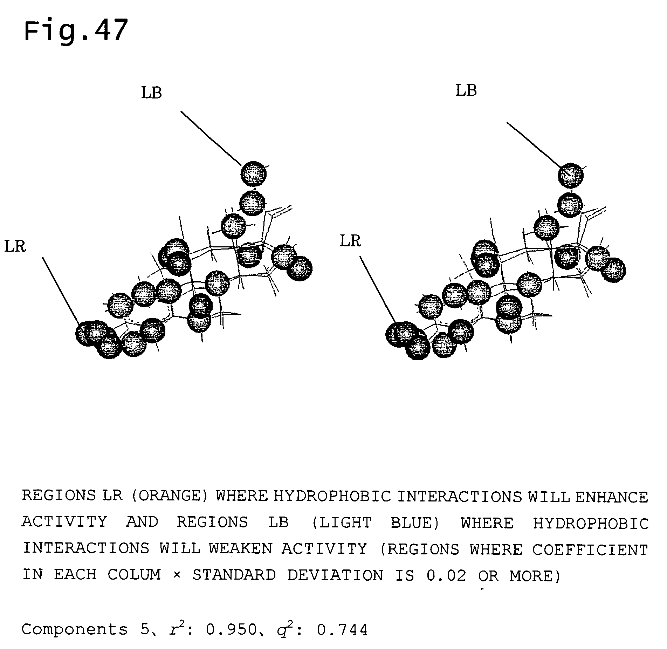

The hydrophobic interactions were studied using the modified Fauchère formula as an attenuation function. Fig. 47 shows the result.

### III-4. 3D QSAR ANALYSIS USING CYCLOOXYGENASE (COX-2) INHIBITORS

Further, as a way to confirm the validity of this method, 3D QSAR analysis was conducted using the 40 cyclooxygenase (COX-2) inhibitors reported by Liu et al. (Liu, H.; Huang, X.; Shen, J.; Luo, X.; Li, M.; Xiong, B.; Chen, G.; Yang, Y.; Jiang, H.; Chen, K. Inhibitory Mode of 1,5-Diarylpyrazole Derivatives Against Cyclooxygenase-2 and Cyclooxygenase-1: Molecular Docking and 3D QSAR Analyses. J. Med. Chem. 2002, 45, 4816-4827). For this analysis, the binding conformations of 1,5-diarylpyrazole derivatives and COX-2 which Liu et al. calculated using AutoDock were used.

During generation of represented points, cluster analysis was conducted without adding a pseudo-atom at the center of a ring. The threshold value for generation of represented points was 0.75 angstrom. As the represented points, 97 points were obtained.

For interactions between the represented points and each atom used in analysis, the combinations of procedures yielding the favorable results among the procedures executed so far were used. In short, 3D QSAR analysis was conducted using two types of procedures, one being use of the SEAL-type attenuation function (10; the combination of 1-B and 4-E to compute the steric, the electrostatic and the hydrophobic interactions) and the other being use of indicator variables (11; the combination of 6-J, 7-K and 4-F to compute the steric, the electrostatic and the hydrophobic interactions).

### (10) USE OF SEAL-TYPE ATTENUATION FUNCTION

To use the SEAL-type Gaussian attenuation function, SEAL parameters were applied as for the steric and the electrostatic interactions and FLEXS parameters were applied as for the hydrophobic interactions. With this approach, a favorable result was not obtained.

### (11) USE OF INDICATOR VARIABLES

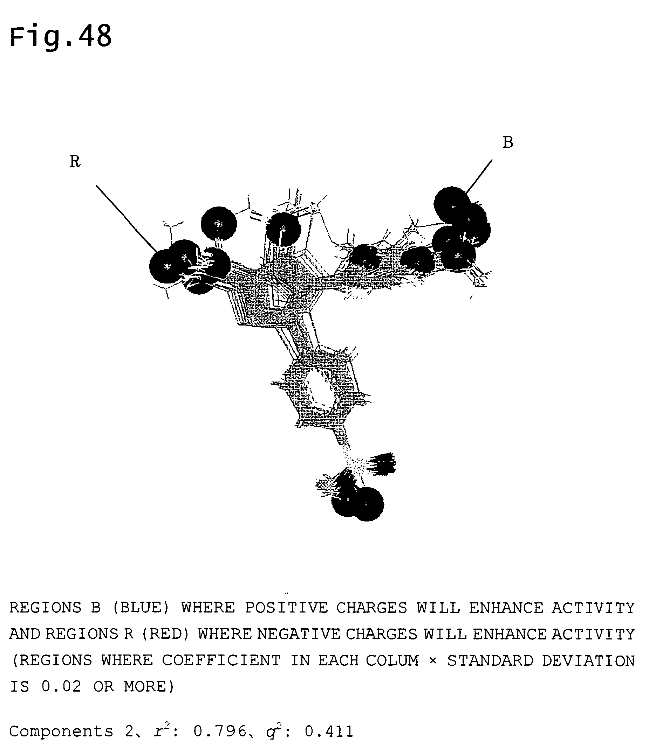

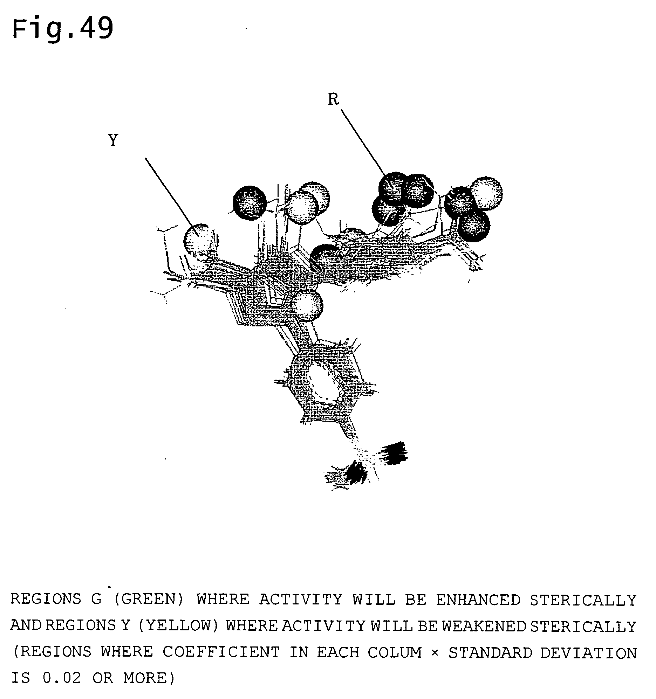

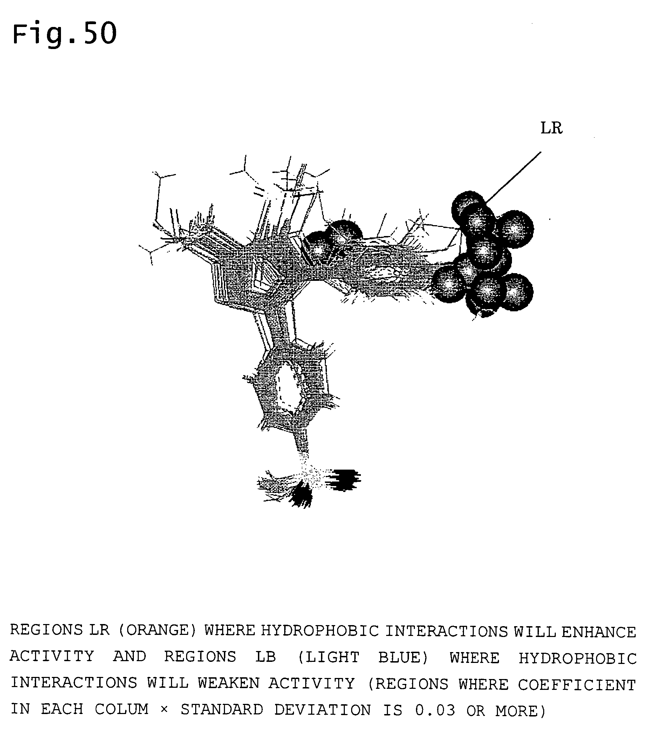

For use of indicator variables, FLEXS parameters were applied as for the hydrophobic interactions. In other words, the combination of 6-J, 7-K and 4-F was used to compute the steric, the electrostatic and the hydrophobic interactions. Figs. 48 through 50 show the computed results. In these drawings, the same color chart to those used in III-1, III-2 and III-3 is used for the respective regions.

### IV. RESULTS AND DISCUSSION

### IV-1. CALCULATION OF STERIC INTERACTIONS AND ELECTROSTATIC INTERACTIONS

Table 2 shows the CoMFA and CoMSIA results according to the present invention. Only the steric contribution is used for QSAR analysis in CoMFA. Meanwhile, in CoMSIA, although precise comparison is impossible since QSAR analysis uses three parameters of the steric, the electrostatic and the hydrophobic terms, q² is the same between CoMFA and CoMSIA while r² is slightly better in CoMSIA.

**Table 2**

| | CoMFA | CoMSI A | 1-A | 1-B | 1-C | 1-D | 2-B | 2-C |
|---|---|---|---|---|---|---|---|---|
| The number of components | 2 | 4 | 2 | 4 | 4 | 4 | 4 | 2 |
| r² | 0.879 | 0.941 | 0.899 | 0.915 | 0.984 | 0.982 | 0.915 | 0.976 |
| q² | 0.662 | 0.662 | 0.760 | 0.528 | 0.822 | 0.798 | 0.521 | 0.741 |
| electrostatic contribution | ----- | 0.086 | ----- | 0.757 | 0.458 | 0.500 | 0.783 | 0.480 |
| steric contribution | 1.000 | 0.535 | 1.000 | 0.243 | 0.542 | 0.500 | 0.217 | 0.520 |
| hydrophobic contribution | ------ | 0.378 | ----- | ----- | ----- | ------ | ----- | ----- |
| Corresponding drawing | | Figs. 5 and 6 | Fig. 10 | Figs. 12 and 13 | Figs. 15 and 16 | Figs. 18 and 19 | Figs. 21 and 22 | Figs. 24 and 25 |

### IV-2. CALCULATION OF HYDROPHOBIC INTERACTIONS

The hydrophobic interactions were studied in the present invention, identifying that a result greatly changed depending upon the combination of hydrophobic parameters and evaluation functions. In other words, where AlogP according to Viswanadhan et al. is applied as a hydrophobic parameter (3-E, 3-F), an evaluation function used in SEAL produces a drawing which shows only a region where hydrophobic interactions will weaken the activity (Fig. 26, 3-E). As compared with the other approaches, r² and q² are inferior (Table 3; Fig. 27, 3-F).

**Table 3**

| | CoMSIA | 3-E | 3-F | 4-E | 4-F | 5-E | 5-F |
|---|---|---|---|---|---|---|---|
| The number of components | 2 | 1 | 2 | 2 | 2 | 1 | 2 |
| r² | 0.795 | 0.568 | 0.879 | 0.666 | 0.722 | 0.881 | 0.810 |
| q² | 0.455 | 0.381 | 0.707 | 0.408 | 0.442 | 0.747 | 0.534 |
| Correspond ing drawing | | Fig.26 | Fig.27 | Fig.28 | Fig.29 | Fig.30 | Fig.31 |

On the contrary, use of indicator variables as an evaluation function results in intertwinement of a region where hydrophobic interactions will enhance the activity and a where hydrophobic interactions will weaken the activity. It is concluded therefore that use of AlogP as a hydrophobic parameter for the method according to the present invention is not appropriate. With application of FLEXS hydrophobic parameters (4-E, 4-F), similar results to a CoMSIA result are obtainable both when an attenuation function for SEAL is used as an evaluation function and when indicator variables are used as an evaluation function. (Figs. 28 and 29, 4-E, 4-F)

With the method according to the present invention, since a large number of coordinate points are generated where the structure changes greatly, the coordinate points are close to each other in a region which is expected to contribute to the activity but are spaced apart from each other in a region which is expected not to largely contribute to the activity. It is therefore assumed that, as compared with CoMSIA, many regions where hydrophobic interactions weaken the activity appear around the 17-position and there are not many activity-weakening regions around the A-ring. Although the result yielded from the indicator variables is somewhat better (Table 3, 4-E vs 4-F), with the both procedures, q² which is reliable as a model is obtained. Where indicator variables are used, both r² and q² are comparable to those obtained in CoMSIA.

### (Table 3, 4-F)

Since HASL parameters are not merely hydrophobic parameters but also parameters containing the electron density, while both r² and q² are higher than in CoMSIA, different drawings are obtained (Fig. 30). That is, when an attenuation function for SEAL is used (5-E), regions where positive HASL parameters will enhance appear the activity around the 3-position and the 17-position side chains, while activity-weakening portions appear at the C-ring side chains. Relatively speaking, it is said that positive HASL parameters contain many atoms which are negatively charged and exhibit hydrophobic interactions with each other and that negative parameters contain many atoms which are negatively charged and exhibit hydrophobic interactions. It then follows that it is possible to enhance the activity by negatively charged atoms exhibiting hydrophobic interactions around the 3-position and the 17-position side chains. However, this result is of the opposite trend to the earlier reports, CoMSIA, etc. This is presumably because HASL parameters are not indicative of simple hydrophobic or electrostatic interactions. Noting this, a review of how strongly HASL parameters reflect which physiochemical parameters in the method according to the present invention will hopefully expand the range of applications of the method according to the present invention.

On the contrary, when indicator variables are used as evaluation functions while applying HASL parameters, a favorable result was not obtained. (Fig. 31)

### IV-3. INFLUENCES OF ATTENUATION FUNCTIONS USED IN CALCULATION OF INTERACTIONS

Study of the application of each attenuation function in the method according to the present invention has not shown any great difference between steric interactions and electrostatic interactions due to a difference of attenuation functions. Use of the SEAL-type Gaussian function (J) produced the highest r² and q², and use of the Fauchère formula (H) yielded the next favorable result. Comparison of regions contributing to the activity shows that while a region where the activity will be enhanced sterically appears around the 17-position methyl group with CoMSIA, (G), (H) and (I), this region does not appear when the SEAL-type Gaussian function (J) is used. Since a contour map does not appear in this region with CoMFA, it may be that this region does not contribute greatly to onset of the activity. With this method as well, an activity-weakening region appears around the 3-position of the A-ring and an activity-enhancing region appears around a steroid side chain. Table 4 shows the result.

**Table 4**

| Influences of the attenuation functions over the steric interactions | | | | | |
|---|---|---|---|---|---|
| Attenuation function | G | H | I | J | K |
| The number of components | 3 | 2 | 2 | 2 | 2 |
| r² | 0.847 | 0.844 | 0.797 | 0.781 | 0.902 |
| q² | 0.715 | 0.725 | 0.698 | 0.624 | 0.806 |
| Threshold value for regions affecting the activity | 0.03 | 0.01 | 0.01 | 0.02 | 0.02 |
| The number of regions enhancing the activity | 18 | 22 | 14 | 12 | 14 |
| The number of regions weakening the activity | 13 | 10 | 12 | 9 | 7 |
| Corresponding drawing | Fig.32 | Fig.33 | Fig.34 | Fig.35 | Fig.36 |

Regions affecting the activity: Coefficient in each column × standard deviation

Approximately the same results were obtained on the electrostatic interactions between all methods. Comparison regarding the electrostatic effect around the 3-position of the A-ring revealed that while regions where positive charges would enhance the activity appeared when CoMSIA was used, regions where negative charges would enhance the activity appeared around regions where positive charges would enhance the activity when the attenuation functions (G) through (K) were used. This suggests that since the spaces between the represented points or lattice points are smaller in the present invention as compared with CoMSIA, finer 3D QSAR analysis is possible. In addition, a difference from the steric interactions, r² was the best when Gaussian function (K) was used, whereas q² was the best when the Fauchère formula (H) was used. Table 5 shows the result. The items in the chart are the same as those in Table 2.

**Table 5**

| Influences of the attenuation functions over the electrostatic interactions | | | | | |
|---|---|---|---|---|---|
| Attenuation function | G | H | I | J | K |
| The number of components | 4 | 4 | 4 | 4 | 6 |
| r² | 0.970 | 0.970 | 0.949 | 0.903 | 0.983 |
| q² | 0.761 | 0.776 | 0.586 | 0.579 | 0.719 |
| Threshold value for regions affecting the activity | 0.03 | 0.03 | 0.03 | 0.04 | 0.03 |
| The number of regions enhancing the activity | 11 | 8 | 19 | 14 | 9 |
| The number of regions weakening the activity | 10 | 10 | 12 | 9 | 12 |
| Corresponding drawing | Fig.37 | Fig.38 | Fig.39 | Fig.40 | Fig.41 |

Regions affecting the activity: Coefficient in each column × standard deviation

Although these attenuation functions are application of the molecular lipophilic potential (MLP) potential function to the present invention, a favorable result was not obtained despite our expectation. MLP was developed originally as a potential function for calculation of hydrophobic interactions, and uses unique parameters such as AlogP which were developed for an logP calculation method and an attenuation parameter. When the AlogP parameter was applied, favorable results were not obtained from the three types of attenuation functions (G), (H) and (I) as it was not possible to obtain a favorable result from the SEAL-type Gaussian function (J). Table 6 shows the result. The items in the chart are the same as those in Table 2.

**Table 6**

| Influences of the attenuation functions applied with AlogP over the hydrophobic interactions | | | | |
|---|---|---|---|---|
| Attenuation function | G | H | I | J |
| The number of components | ----- | 2 | 2 | 2 |
| r² | ----- | 0.700 | 0.612 | 0.666 |
| q² | ----- | 0.254 | 0.171 | 0.408 |
| Threshold value for regions affecting the activity | ----- | 0.02 | 0.02 | 0.02 |
| The number of regions enhancing the activity | ----- | 7 | 6 | 3 |
| The number of regions weakening the activity | ----- | 16 | 17 | 16 |
| Corresponding drawing | | Fig.42 | Fig.43 | Fig.44 |

Regions affecting the activity: Coefficient in each column × standard deviation

The electrostatic interactions were studied applying FLEXS parameters, and it was not possible to obtain an optimal QSAR model from the Audry formula (G). While regions where hydrophobic interactions would enhance the activity and regions where hydrophobic interactions would weaken the activity were approximately the same between H and J, and r² and q² were the best respectively with the Fauchère formula (H) and the SEAL-type Gaussian function (J). Table 7 shows the result. The items in the chart are the same as those in Table 2.

**Table 7**

| Influences of the attenuation functions applied with FLEXS parameters over the hydrophobic interactions | | | |
|---|---|---|---|
| Attenuation function | G | H | I |
| The number of components | 4 | 3 | 5 |
| r² | 0.934 | 0.924 | 0.950 |
| q² | 0.705 | 0.741 | 0.744 |
| Threshold value for regions affecting the activity | 0.03 | 0.03 | 0.05 |
| The number of regions enhancing the activity | 12 | 7 | 10 |
| The number of regions weakening the activity | 16 | 14 | 11 |
| Corresponding drawing | Fig.45 | Fig.46 | Fig.47 |

Regions affecting the activity: Coefficient in each column × standard deviation

An overall result of the above is that the SEAL-type Gaussian function or the Fauchère formula (FORMULA 2) are appropriate for the present invention.

### IV-4. 3D QSAR ANALYSIS USING CYCLOOXYGENASE (COX-2) INHIBITORS

Although the method (10) did not produce a favorable result, q² and r² were sufficiently high when the method (11) was used. Soliva et al. have defined partial structures in (i) 5-ring portions (5MR), (ii) benzene ring (SR) portions substituted with sulfone/sulfonamide and (iii) other substitutional groups or unsubstituted benzene (BR) portions and reported the relationships between the structures and activity (Solvia, Rl; Almansa, C.; Kalko, S.G.; Luque, F. J.; Orozco, M. Theoretical Studies on the Inhibition Mechanism of Cyclooxygenase-2. Is There a Unique Recognition Site? J. Med. Chem. 2003, 46, 1372-1382). Around BR portions, regions where steric interactions would enhance the activity appeared in a considerably different way from the analysis results according to Soliva et al.

Although this may seem different from the CoMFA and CoMSIA results, it is inferred that while CoMFA and CoMSIA reflect the circumstance around the molecules, the method according to the present invention is directed to calculation of the interactions in the portions occupied with the molecules and is characterized in greatly reflecting which sections of the molecules strongly influence the activity.

**Table 8**

| 3D QSAR analysis on COX-2 inhibitors | | |
|---|---|---|
| | 10 | 11 |
| r² | 0.144 | 0.411 |
| q² | 0.675 | 0.796 |
| The number of components | 3 | 2 |
| electrostatic contribution rate | 0.478 | 0.379 |
| steric contribution rate | 0.156 | 0.244 |
| hydrophobic contribution rate | 0.366 | 0.377 |
| Corresponding drawing | ----- | Figs48,49,50 |

On the contrary, the sections around 5MR where the steric interactions were not desirable for the activity were similar to those obtained with CoMFA and CoMSIA.

Sterically undesirable regions may appear around desirable regions according to the present invention, which allows identification of specific candidates for molecular synthesis at a better accuracy than CoMFA and CoMSIA.

Evaluation functions to use may be any evaluation formulae besides the known evaluation formulae described above. Among the evaluation formulae studied by the inventors of the present invention, use of the SEAL-type evaluation formula (1-B) and use of the indicator variables (1-D) are applicable to efficient drug design as methods of providing a convenient and favorable 3D QSAR method which can run on a standard PC.

## Claims

1. A three-dimensional quantitative structure-activity relationship method of extracting and visually displaying characteristics of a compound based on the atomic coordinates of plural molecules superposed within a virtual space, comprising:
a process A of superposing plural molecules in a virtual space;
a process B of performing cluster analysis of the atomic coordinates of said plural molecules thus superposed in said virtual space and thereby generating represented points;
a process C of calculating interactions between the respective atoms of said plural molecules thus superposed and said represented points; and
a process D of statistically analyzing said interactions,
wherein said process B of cluster analysis further comprises:
a first process B1 of calculating the coordinates of the respective atoms contained in said plural molecules thus superposed in said virtual space;
a second process B2 of calculating interatomic distances between each atom and other atoms and identifying the shortest interatomic distance among thus calculated interatomic distances and two atoms constituting the shortest interatomic distance;
a third process B3 of deleting said two atoms having the shortest interatomic distance from said three-dimensional space and generating an atom which represents said two atoms in the weighted average coordinates of said two atoms to delete, when the shortest interatomic distance thus calculated is equal to or smaller than a predetermined threshold value;
a fourth process B4 of returning to said second process B2 after said third process B3 and executing said second process B2 including said atoms formed during said third process B3; and
a fifth process B5 of terminating said process B when the shortest interatomic distance thus calculated is exceeds said predetermined threshold.

2. A three-dimensional quantitative structure-activity relationship method of extracting and visually displaying characteristics of a compound based on the atomic coordinates of plural molecules superposed within a virtual space, comprising:
a process A of superposing plural molecules in a virtual space;
a process B of performing cluster analysis of the atomic coordinates of said plural molecules thus superposed in said virtual space and thereby generating represented points;
a process C of calculating interactions between the respective atoms of said plural molecules thus superposed and said represented points; and
a process D of statistically analyzing said interactions,
wherein said process B of cluster analysis further comprises:
a process B1 of, when said molecules thus superposed in said virtual space include a ring structure or functional group, generating an imaginary atom at a position representing said ring structure or functional group when needed;
a process B2 of calculating interatomic distances between each atom and other atoms as for all atoms in said virtual space including said imaginary atom and identifying the shortest interatomic distance among thus calculated interatomic distances and two atoms constituting the shortest interatomic distance;
a process B3 of deleting said two atoms having the shortest interatomic distance from said three-dimensional space and generating an atom which represents said two atoms in the weighted average coordinates of said two atoms to delete, when thus calculated shortest interatomic distance is equal to or smaller than a predetermined threshold value;
a fourth process B4 of returning to said second process B2 after said third process B3 and executing said second process B2 including said atoms formed during said third process B3; and
a fifth process B5 of terminating said process B when the shortest interatomic distance thus calculated is exceeds said predetermined threshold.

3. The three-dimensional quantitative structure-activity relationship method of claim 1 or 2, wherein said interactions calculated during said process C include at least one of steric interactions, electrostatic interactions and hydrophobic interactions.

4. A program for a three-dimensional quantitative structure-activity relationship method of extracting and visually displaying characteristics of a compound based on the atomic coordinates of plural molecules superposed within a virtual space, said program making a computer execute:
a process A of superposing plural molecules in a virtual space;
a process B of performing cluster analysis of the atomic coordinates of said plural molecules thus superposed in said virtual space and thereby generating represented points;
a process C of calculating interactions between the respective atoms of said plural molecules thus superposed and the represented points; and
a process D of statistically analyzing said interactions,
wherein said process B of cluster analysis further comprises:
a first process B1 of calculating the coordinates of the respective atoms contained in said plural molecules thus superposed in said virtual space;
a second process B2 of calculating interatomic distances between each atom and other atoms and identifying the shortest interatomic distance among thus calculated interatomic distances and two atoms constituting the shortest interatomic distance;
a third process B3 of deleting said two atoms having the shortest interatomic distance from said three-dimensional space and generating an atom which represents said two atoms in the weighted average coordinates of said two atoms to delete, when thus calculated shortest interatomic distance is equal to or smaller than a predetermined threshold value;
a fourth process B4 of returning to said second process B2 after said third process B3 and executing said second process B2 including said atoms formed during said third process B3; and
a fifth process B5 of terminating said process B when thus calculated shortest interatomic distance is exceeds said predetermined threshold.

5. A program for a three-dimensional quantitative structure-activity relationship method of extracting and visually displaying characteristics of a compound based on the atomic coordinates of plural molecules superposed within a virtual space, said program making a computer execute:
a process A of superposing plural molecules in a virtual space;
a process B of performing cluster analysis of the atomic coordinates of said plural molecules thus superposed in said virtual space and thereby generating represented points;
a process C of calculating interactions between the respective atoms of said plural molecules thus superposed and the represented points; and
a process D of statistically analyzing said interactions,
wherein said process B of cluster analysis further comprises:
a first process B1 of, when said molecules thus superposed in said virtual space include a ring structure or functional group, generating an imaginary atom at a position which represents said ring structure or functional group when needed;
a second process B2 of, as for all atoms in said virtual space including said imaginary atom, calculating interatomic distances with other atoms and identifying the shortest interatomic distance among thus calculated interatomic distances and two atoms constituting the shortest interatomic distance;
a third process B3 of deleting said two atoms having the shortest interatomic distance from said three-dimensional space and generating an atom which represents said two atoms in the weighted average coordinates of said two atoms to delete, when thus calculated shortest interatomic distance is equal to or smaller than a predetermined threshold value;
a fourth process B4 of returning to said second process B2 after said third process B3 and executing said second process B2 including said atoms formed during said third process B3; and
a fifth process B5 of terminating said process B when thus calculated shortest interatomic distance is exceeds said predetermined threshold.

6. The program of claim 4 or 5, wherein said interactions calculated during said process C include at least one of steric interactions, electrostatic interactions and hydrophobic interactions.
